# EUROPEAN PATENT APPLICATION

(11) **EP 4 796 535 A1**
(43) Date of publication of application: **26.08.2026**
(21) Application number: 24880077.3
(22) Date of filing: 15.10.2024
(51) Int. Cl.: C07D 401/04, A61K 31/454, A61P 35/00

(54) **NOVEL MOLECULAR ADHESIVE AND USE THEREOF**

(30) Priority: 16.10.2023 KR 20230137434
(71) Applicant: Astrogen, Inc., Daegu 41072 (KR)
(72) Inventor: HWANG, Su-Kyeong, Daegu 41072 (KR); RYU, Hyung Chul, Daegu 41072 (KR)
(74) Representative: Dehns
(86) International application number: PCT/KR2024/015570
(87) International publication number: WO 2025/084740

(57) **Abstract**

The present invention relates to a novel compound capable of functioning as a molecular adhesive, or an enantiomeric compound thereof, and a preparation method therefor and use thereof.

## Description

### Technical Field

The present invention relates to a novel compound or an enantiomer thereof which can function as a molecular glue, and a method for preparing the same and a use thereof.

### Background Art

Targeted protein degradation (TPD) refers to a mechanism in which after a disease-causing factor has been translated into a protein, the corresponding protein is directly degraded and eliminated, and is represented by proteolysis-targeting chimeras (PROTAC). PROTAC consists of a protein of interest (POI) binding moiety (corresponding to the substrate protein linking region), a linker, and an E3 ligase binding moiety (corresponding to the enzyme linking region), and the core of PROTAC technology is to allow the corresponding substrate of the TPD mechanism to directly meet the E3 ligase enzyme. PROTAC consists of a protein of interest (POI) binding moiety (corresponding to a substrate protein linking region), a linker, and an E3 ligase binding moiety (corresponding to an enzyme linking region), and the core of the PROTAC technology is to allow the corresponding substrate of the TPD mechanism to directly meet the E3 ligase enzyme.

Molecular glues, which are small molecules with properties different from PROTACs, can interact with a variety of target proteins that are difficult to predict. PROTACs are divalent molecules consisting of two parts, in which one part binds to a POI and the other part binds to an E3 ligase. While PROTACs are connected by linkers and have relatively large molecular weights, molecular glues are smaller than PROTACs, and are advantageous in that they have superior pharmacological properties, higher membrane permeability, and a superior cellular uptake rate compared to PROTACs.

Various types of molecular glues have been discovered, and representative examples include thalidomide-related analogues, and lenalidomide and pomalidomide, which target the E3 ligase cereblon (CRBN).

Safety and tolerability are important considerations in the development of anticancer agents, thus raising the need for the development of immunomodulators with a favorable safety profile while minimizing side effects and long-term complications. Traditional cancer treatments, particularly chemotherapy and targeted therapies, work by attacking tumor cells. Since chemotherapy drugs (e.g., cisplatin and doxorubicin) primarily target rapidly dividing cells, they affect normal cells as well as cancer cells, thereby causing serious side effects. Targeted therapies (e.g., imatinib which is a tyrosine kinase inhibitor) block cancer growth by inhibiting specific proteins, but these therapies have a problem in that drug resistance may develop with long-term use.

Since molecular glues work by removing the target protein itself rather than inhibiting its function, they have a high potential to overcome the problem of mutational resistance. In addition, since molecular glues achieve fundamental removal of the target protein rather than simple inhibition of the protein, it is possible to maintain a continuous therapeutic effect, and since it does not require a high dose of a drug, molecular glues may be less toxic and safer than existing anticancer agents.

### Disclosure of Invention

### Technical Problem

An object of the present invention is to provide a novel compound capable of functioning as a molecular glue, or an enantiomeric compound thereof. In addition, the compound may be used for the prevention or treatment of cancer, and more specifically, an object of the present invention is to provide use of the compound for the prevention or treatment of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.

### Solution to Problem

[1] In an aspect of the present invention, the present invention relates to a compound represented by the following Formula 1, or an enantiomeric compound thereof: 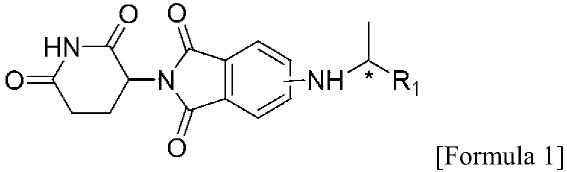
   where, the R₁ is alkyl, saturated or unsaturated cycloalkyl, aromatic or non-aromatic heterocyclyl, aryl, or -alkyl-C(=O)-O-alkyl,
   the alkyl is unsubstituted or substituted with one or more alkoxy groups or hydroxy groups, and
   the heterocyclyl or aryl is unsubstituted or substituted with one or more alkyl groups, hydroxy groups, halogens, alkoxy groups, -(C=O)-O-alkyl, -CF₃, -OCF₃, -S-alkyl, -S(O₂)-alkyl, or -S(O₂)NH₂.
[2] In [1] above, the R₁ may be C₁₋₇ alkyl, C₃₋₇ cycloalkyl, 5- to 12-membered aromatic or non-aromatic heterocyclyl including a heteroatom selected from the group consisting of N, O, and S, or C₆₋₁₂ aryl.
[3] In [1] above, the R₁ may be substituted or unsubstituted C₁₋₅ alkyl, substituted or unsubstituted C₃₋₅ cycloalkyl, substituted or unsubstituted dihydrobenzofuran or pyridine, substituted or unsubstituted phenyl, or C₁₋₅ alkyl-(C=O)-O-alkyl.
[4] In [1] above, the R₁ may be C₁₋₃ alkyl substituted with a hydroxy group; unsubstituted C₃₋₅ alkyl; or a phenyl substituted with a methoxy group .
[5] In [1] above, the compound represented by Formula 1 above may be any one selected from the group consisting of the following:
   1) methyl (3R)-3-((2-(2,6-dioxopiperidin-3-yl)-1,3-dioxoisoindolin-4-yl)amino)butanoate;
   2) methyl (3S)-3-((2-(2,6-dioxopiperidin-3-yl)-1,3-dioxoisoindolin-4-yl)amino)butanoate;
   3) 2-(2,6-dioxopiperidin-3-yl)-4-(((S)-1-hydroxypropan-2-yl)amino)isoindoline-1,3-dione;
   4) 2-(2,6-dioxopiperidin-3-yl)-4-(((R)-1-hydroxypropan-2-yl)amino)isoindoline-1,3-dione;
   5) 2-(2,6-dioxopiperidin-3-yl)-4-(((S)-pentan-2-yl)amino)isoindoline-1,3-dione;
   6) 2-(2,6-dioxopiperidin-3-yl)-4-((3-methylbutan-2-yl)amino)isoindoline-1,3-dione;
   7) 4-((3,3-dimethylbutan-2-yl)amino)-2-(2,6-dioxopiperidin-3-yl)isoindoline-1,3-dione;
   8) 2-(2,6-dioxopiperidin-3-yl)-4-(((S)-1-methoxypropan-2-yl)amino)isoindoline-1,3-dione;
   9) 2-(2,6-dioxopiperidin-3-yl)-4-(((R)-1-(4-fluorophenyl)ethyl)amino)isoindoline-1,3-dione;
   10) 2-(2,6-dioxopiperidin-3-yl)-4-(((S)-1-(4-fluorophenyl)ethyl)amino)isoindoline-1,3-dione;
   11) 2-(2,6-dioxopiperidin-3-yl)-4-(((S)-1-(4-methoxyphenyl)ethyl)amino)isoindoline-1,3-dione;
   12) 2-(2,6-dioxopiperidin-3-yl)-4-(((R)-1-(4-methoxyphenyl)ethyl)amino)isoindoline-1,3-dione;
   13) 2-(2,6-dioxopiperidin-3-yl)-4-(((R)-1-(4-(trifluoromethoxy)phenyl)ethyl)amino)isoindoline-1,3-dione;
   14) 2-(2,6-dioxopiperidin-3-yl)-4-(((S)-1-(4-(trifluoromethoxy)phenyl)ethyl)amino)isoindoline-1,3-dione;
   15) 2-(2,6-dioxopiperidin-3-yl)-4-(((R)-1-(2-fluorophenyl)ethyl)amino)isoindoline-1,3-dione;
   16) 2-(2,6-dioxopiperidin-3-yl)-4-(((S)-1-(2-fluorophenyl)ethyl)amino)isoindoline-1,3-dione;
   17) 2-(2,6-dioxopiperidin-3-yl)-4-(((R)-1-(3-methoxyphenyl)ethyl)amino)isoindoline-1,3-dione;
   18) 2-(2,6-dioxopiperidin-3-yl)-4-(((S)-1-(3-methoxyphenyl)ethyl)amino)isoindoline-1,3-dione;
   19) 2-(2,6-dioxopiperidin-3-yl)-4-(((R)-1-(2-(trifluoromethyl)phenyl)ethyl)amino)isoindoline-1,3-dione;
   20) 4-((1R)-1-((2-(2,6-dioxopiperidin-3-yl)-1,3-dioxoisoindolin-4-yl)amino)ethyl)benzenesulfonamide;
   21) methyl 4-((1R)-1-((2-(2,6-dioxopiperidin-3-yl)-1,3-dioxoisoindolin-4-yl)amino)ethyl)benzoate;
   22) methyl 4-((1S)-1-((2-(2,6-dioxopiperidin-3-yl)-1,3-dioxoisoindolin-4-yl)amino)ethyl)benzoate;
   23) 2-(2,6-dioxopiperidin-3-yl)-4-(((R)-1-(p-tolyl)ethyl)amino)isoindoline-1,3-dione;
   24) 2-(2,6-dioxopiperidin-3-yl)-4-(((S)-1-(p-tolyl)ethyl)amino)isoindoline-1,3-dione;
   25) 4-(((R)-1-(4-chlorophenyl)ethyl)amino)-2-(2,6-dioxopiperidin-3-yl)isoindoline-1,3-dione;
   26) 4-(((S)-1-(4-chlorophenyl)ethyl)amino)-2-(2,6-dioxopiperidin-3-yl)isoindoline-1,3-dione;
   27) 2-(2,6-dioxopiperidin-3-yl)-4-(((R)-1-(2-fluoro-4-methoxyphenyl)ethyl)amino)isoindoline-1,3-dione;
   28) 2-(2,6-dioxopiperidin-3-yl)-4-(((S)-1-(2-fluoro-4-methoxyphenyl)ethyl)amino)isoindoline-1,3-dione;
   29) 2-(2,6-dioxopiperidin-3-yl)-4-(((R)-1-(4-fluoro-3-methoxyphenyl)ethyl)amino)isoindoline-1,3-dione;
   30) 2-(2,6-dioxopiperidin-3-yl)-4-(((S)-1-(4-fluoro-3-methoxyphenyl)ethyl)amino)isoindoline-1,3-dione;
   31) 2-(2,6-dioxopiperidin-3-yl)-4-(((R)-1-(4-(trifluoromethyl)phenyl)ethyl)amino)isoindoline-1,3-dione;
   32) 2-(2,6-dioxopiperidin-3-yl)-4-(((S)-1-(4-(trifluoromethyl)phenyl)ethyl)amino)isoindoline-1,3-dione;
   33) 2-(2,6-dioxopiperidin-3-yl)-4-(((R)-1-(4-(methylthio)phenyl)ethyl)amino)isoindoline-1,3-dione;
   34) 2-(2,6-dioxopiperidin-3-yl)-4-(((S)-1-(4-(methylthio)phenyl)ethyl)amino)isoindoline-1,3-dione;
   35) 2-(2,6-dioxopiperidin-3-yl)-4-(((S)-1-(4-ethoxyphenyl)ethyl)amino)isoindoline-1,3-dione;
   36) 2-(2,6-dioxopiperidin-3-yl)-4-(((R)-1-(3-fluorophenyl)ethyl)amino)isoindoline-1,3-dione;
   37) 2-(2,6-dioxopiperidin-3-yl)-4-(((S)-1-(3-fluorophenyl)ethyl)amino)isoindoline-1,3-dione;
   38) 2-(2,6-dioxopiperidin-3-yl)-4-(((R)-1-(3-fluoro-4-methoxyphenyl)ethyl)amino)isoindoline-1,3-dione;
   39) 4-(((R)-1-(3,4-difluorophenyl)ethyl)amino)-2-(2,6-dioxopiperidin-3-yl)isoindoline-1,3-dione;
   40) 4-(((S)-1-(3,4-difluorophenyl)ethyl)amino)-2-(2,6-dioxopiperidin-3-yl)isoindoline-1,3-dione;
   41) 4-(((R)-1-(3,5-difluorophenyl)ethyl)amino)-2-(2,6-dioxopiperidin-3-yl)isoindoline-1,3-dione;
   42) 4-(((S)-1-(3,5-difluorophenyl)ethyl)amino)-2-(2,6-dioxopiperidin-3-yl)isoindoline-1,3-dione;
   43) 2-(2,6-dioxopiperidin-3-yl)-4-(((R)-1-(6-methoxypyridin-3-yl)ethyl)amino)isoindoline-1,3-dione;
   44) 2-(2,6-dioxopiperidin-3-yl)-4-(((S)-1-(6-methoxypyridin-3-yl)ethyl)amino)isoindoline-1,3-dione;
   45) 4-(((R)-1-(3,5-dimethoxyphenyl)ethyl)amino)-2-(2,6-dioxopiperidin-3-yl)isoindoline-1,3-dione;
   46) 2-(2,6-dioxopiperidin-3-yl)-4-(((S)-1-(3-fluoro-4-methoxyphenyl)ethyl)amino)isoindoline-1,3-dione;
   47) 2-(2,6-dioxopiperidin-3-yl)-4-(((R)-1-(5-methoxypyridin-2-yl)ethyl)amino)isoindoline-1,3-dione;
   48) 2-(2,6-dioxopiperidin-3-yl)-4-(((S)-1-(5-methoxypyridin-2-yl)ethyl)amino)isoindoline-1,3-dione;
   49) 2-(2,6-dioxopiperidin-3-yl)-4-(((R)-1-(4-(methylsulfonyl)phenyl)ethyl)amino)isoindoline-1,3-dione;
   50) 2-(2,6-dioxopiperidin-3-yl)-4-(((S)-1-(4-(methylsulfonyl)phenyl)ethyl)amino)isoindoline-1,3-dione;
   51) 4-(((R)-1-(2,3-dihydrobenzofuran-5-yl)ethyl)amino)-2-(2,6-dioxopiperidin-3-yl)isoindoline-1,3-dione; and
   52) 4-(((S)-1-cyclopropylethyl)amino)-2-(2,6-dioxopiperidin-3-yl)isoindoline-1,3-dione.
[6] In [1] above, the compound represented by Formula 1 above may be 2-(2,6-dioxopiperidin-3-yl)-4-(((R)-1-hydroxypropan-2-yl)amino)isoindoline-1,3-dione; 2-(2,6-dioxopiperidin-3-yl)-4-(((S)-pentan-2-yl)amino)isoindoline-1,3-dione; or 2-(2,6-dioxopiperidin-3-yl)-4-(((R)-1-(4-methoxyphenyl)ethyl)amino)isoindoline-1,3-dione.
[7] In another aspect of the present invention, the present invention relates to a pharmaceutical composition for preventing or treating cancer, containing a compound represented by Formula 1 of [1] above, or an enantiomeric compound thereof as an active ingredient,
   where the cancer is any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.
[8] In [7] above, the cancer may be recurrent or incurable cancer.
[9] In [7] above, the cancer may be a drug-resistant cancer.
[10] In [7] above, the active ingredient may be any one selected from the group consisting of the following:
   1) methyl (3R)-3-((2-(2,6-dioxopiperidin-3-yl)-1,3-dioxoisoindolin-4-yl)amino)butanoate;
   2) methyl (3S)-3-((2-(2,6-dioxopiperidin-3-yl)-1,3-dioxoisoindolin-4-yl)amino)butanoate;
   3) 2-(2,6-dioxopiperidin-3-yl)-4-(((S)-1-hydroxypropan-2-yl)amino)isoindoline-1,3-dione;
   4) 2-(2,6-dioxopiperidin-3-yl)-4-(((R)-1-hydroxypropan-2-yl)amino)isoindoline-1,3-dione;
   5) 2-(2,6-dioxopiperidin-3-yl)-4-(((S)-pentan-2-yl)amino)isoindoline-1,3-dione;
   6) 2-(2,6-dioxopiperidin-3-yl)-4-((3-methylbutan-2-yl)amino)isoindoline-1,3-dione;
   7) 4-((3,3-dimethylbutan-2-yl)amino)-2-(2,6-dioxopiperidin-3-yl)isoindoline-1,3-dione;
   8) 2-(2,6-dioxopiperidin-3-yl)-4-(((S)-1-methoxypropan-2-yl)amino)isoindoline-1,3-dione;
   9) 2-(2,6-dioxopiperidin-3-yl)-4-(((R)-1-(4-fluorophenyl)ethyl)amino)isoindoline-1,3-dione;
   10) 2-(2,6-dioxopiperidin-3-yl)-4-(((S)-1-(4-fluorophenyl)ethyl)amino)isoindoline-1,3-dione;
   11) 2-(2,6-dioxopiperidin-3-yl)-4-(((S)-1-(4-methoxyphenyl)ethyl)amino)isoindoline-1,3-dione;
   12) 2-(2,6-dioxopiperidin-3-yl)-4-(((R)-1-(4-methoxyphenyl)ethyl)amino)isoindoline-1,3-dione;
   13) 2-(2,6-dioxopiperidin-3-yl)-4-(((R)-1-(4-(trifluoromethoxy)phenyl)ethyl)amino)isoindoline-1,3-dione;
   14) 2-(2,6-dioxopiperidin-3-yl)-4-(((S)-1-(4-(trifluoromethoxy)phenyl)ethyl)amino)isoindoline-1,3-dione;
   15) 2-(2,6-dioxopiperidin-3-yl)-4-(((R)-1-(2-fluorophenyl)ethyl)amino)isoindoline-1,3-dione;
   16) 2-(2,6-dioxopiperidin-3-yl)-4-(((S)-1-(2-fluorophenyl)ethyl)amino)isoindoline-1,3-dione;
   17) 2-(2,6-dioxopiperidin-3-yl)-4-(((R)-1-(3-methoxyphenyl)ethyl)amino)isoindoline-1,3-dione;
   18) 2-(2,6-dioxopiperidin-3-yl)-4-(((S)-1-(3-methoxyphenyl)ethyl)amino)isoindoline-1,3-dione;
   19) 2-(2,6-dioxopiperidin-3-yl)-4-(((R)-1-(2-(trifluoromethyl)phenyl)ethyl)amino)isoindoline-1,3-dione;
   20) 4-((1R)-1-((2-(2,6-dioxopiperidin-3-yl)-1,3-dioxoisoindolin-4-yl)amino)ethyl)benzenesulfonamide;
   21) methyl 4-((1R)-1-((2-(2,6-dioxopiperidin-3-yl)-1,3-dioxoisoindolin-4-yl)amino)ethyl)benzoate;
   22) methyl 4-((1S)-1-((2-(2,6-dioxopiperidin-3-yl)-1,3-dioxoisoindolin-4-yl)amino)ethyl)benzoate;
   23) 2-(2,6-dioxopiperidin-3-yl)-4-(((R)-1-(p-tolyl)ethyl)amino)isoindoline-1,3-dione;
   24) 2-(2,6-dioxopiperidin-3-yl)-4-(((S)-1-(p-tolyl)ethyl)amino)isoindoline-1,3-dione;
   25) 4-(((R)-1-(4-chlorophenyl)ethyl)amino)-2-(2,6-dioxopiperidin-3-yl)isoindoline-1,3-dione;
   26) 4-(((S)-1-(4-chlorophenyl)ethyl)amino)-2-(2,6-dioxopiperidin-3-yl)isoindoline-1,3-dione;
   27) 2-(2,6-dioxopiperidin-3-yl)-4-(((R)-1-(2-fluoro-4-methoxyphenyl)ethyl)amino)isoindoline-1,3-dione;
   28) 2-(2,6-dioxopiperidin-3-yl)-4-(((S)-1-(2-fluoro-4-methoxyphenyl)ethyl)amino)isoindoline-1,3-dione;
   29) 2-(2,6-dioxopiperidin-3-yl)-4-(((R)-1-(4-fluoro-3-methoxyphenyl)ethyl)amino)isoindoline-1,3-dione;
   30) 2-(2,6-dioxopiperidin-3-yl)-4-(((S)-1-(4-fluoro-3-methoxyphenyl)ethyl)amino)isoindoline-1,3-dione;
   31) 2-(2,6-dioxopiperidin-3-yl)-4-(((R)-1-(4-(trifluoromethyl)phenyl)ethyl)amino)isoindoline-1,3-dione;
   32) 2-(2,6-dioxopiperidin-3-yl)-4-(((S)-1-(4-(trifluoromethyl)phenyl)ethyl)amino)isoindoline-1,3-dione;
   33) 2-(2,6-dioxopiperidin-3-yl)-4-(((R)-1-(4-(methylthio)phenyl)ethyl)amino)isoindoline-1,3-dione;
   34) 2-(2,6-dioxopiperidin-3-yl)-4-(((S)-1-(4-(methylthio)phenyl)ethyl)amino)isoindoline-1,3-dione;
   35) 2-(2,6-dioxopiperidin-3-yl)-4-(((S)-1-(4-ethoxyphenyl)ethyl)amino)isoindoline-1,3-dione;
   36) 2-(2,6-dioxopiperidin-3-yl)-4-(((R)-1-(3-fluorophenyl)ethyl)amino)isoindoline-1,3-dione;
   37) 2-(2,6-dioxopiperidin-3-yl)-4-(((S)-1-(3-fluorophenyl)ethyl)amino)isoindoline-1,3-dione;
   38) 2-(2,6-dioxopiperidin-3-yl)-4-(((R)-1-(3-fluoro-4-methoxyphenyl)ethyl)amino)isoindoline-1,3-dione;
   39) 4-(((R)-1-(3,4-difluorophenyl)ethyl)amino)-2-(2,6-dioxopiperidin-3-yl)isoindoline-1,3-dione;
   40) 4-(((S)-1-(3,4-difluorophenyl)ethyl)amino)-2-(2,6-dioxopiperidin-3-yl)isoindoline-1,3-dione;
   41) 4-(((R)-1-(3,5-difluorophenyl)ethyl)amino)-2-(2,6-dioxopiperidin-3-yl)isoindoline-1,3-dione;
   42) 4-(((S)-1-(3,5-difluorophenyl)ethyl)amino)-2-(2,6-dioxopiperidin-3-yl)isoindoline-1,3-dione;
   43) 2-(2,6-dioxopiperidin-3-yl)-4-(((R)-1-(6-methoxypyridin-3-yl)ethyl)amino)isoindoline-1,3-dione;
   44) 2-(2,6-dioxopiperidin-3-yl)-4-(((S)-1-(6-methoxypyridin-3-yl)ethyl)amino)isoindoline-1,3-dione;
   45) 4-(((R)-1-(3,5-dimethoxyphenyl)ethyl)amino)-2-(2,6-dioxopiperidin-3-yl)isoindoline-1,3-dione;
   46) 2-(2,6-dioxopiperidin-3-yl)-4-(((S)-1-(3-fluoro-4-methoxyphenyl)ethyl)amino)isoindoline-1,3-dione;
   47) 2-(2,6-dioxopiperidin-3-yl)-4-(((R)-1-(5-methoxypyridin-2-yl)ethyl)amino)isoindoline-1,3-dione;
   48) 2-(2,6-dioxopiperidin-3-yl)-4-(((S)-1-(5-methoxypyridin-2-yl)ethyl)amino)isoindoline-1,3-dione;
   49) 2-(2,6-dioxopiperidin-3-yl)-4-(((R)-1-(4-(methylsulfonyl)phenyl)ethyl)amino)isoindoline-1,3-dione;
   50) 2-(2,6-dioxopiperidin-3-yl)-4-(((S)-1-(4-(methylsulfonyl)phenyl)ethyl)amino)isoindoline-1,3-dione;
   51) 4-(((R)-1-(2,3-dihydrobenzofuran-5-yl)ethyl)amino)-2-(2,6-dioxopiperidin-3-yl)isoindoline-1,3-dione; and
   52) 4-(((S)-1-cyclopropylethyl)amino)-2-(2,6-dioxopiperidin-3-yl)isoindoline-1,3-dione.
[11] In [7] above, the active ingredient may be 2-(2,6-dioxopiperidin-3-yl)-4-(((R)-1-hydroxypropan-2-yl)amino)isoindoline-1,3-dione; 2-(2,6-dioxopiperidin-3-yl)-4-(((S)-pentan-2-yl)amino)isoindoline-1,3-dione; or 2-(2,6-dioxopiperidin-3-yl)-4-(((R)-1-(4-methoxyphenyl)ethyl)amino)isoindoline-1,3-dione.
[12] In [7] above, the composition may be administered by intraperitoneal administration, intravenous administration, intramuscular administration, subcutaneous administration, intradermal injection, oral administration, topical administration, intranasal injection, intrapulmonary injection, or rectal administration.
[13] In still another aspect of the present invention, the present invention relates to a method for preventing or treating cancer in a subject, which includes administering, to a subject, a compound represented by Formula 1 of [1] above, or an enantiomeric compound thereof,
   where the cancer is any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.
[14] In still another aspect of the present invention, the present invention relates to a use of a compound represented by Formula 1 of [1] above, or an enantiomeric compound thereof, for the preparation of a medicament for preventing or treating cancer,
   where the cancer is any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.
[15] In still another aspect of the present invention, the present invention relates to a use of a compound represented by Formula 1 of [1] above, or an enantiomeric compound thereof, for preventing or treating cancer,
   where the cancer is any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.

### Advantageous Effects of Invention

The present invention relates to a novel compound which can function as a molecular glue, or an enantiomeric compound thereof, and the compound can be effectively used as a molecular adhesive to prevent or treat cancer while minimizing side effects and long-term complications.

### Brief Description of Drawings

Fig. 1 shows the Ames Test results of the compounds in the examples of the present invention in strains TA98 and TA100.
Fig. 2 shows the results of the hERG-related cardiotoxicity test and ligand binding assay of the compounds in the examples of the present invention.
Fig. 3 shows the changes in body weight when the compounds in the examples of the present invention were injected in the MTD test.
Fig. 4 shows the cell killing effect of the compounds in the examples of the present invention in hepatoma cell lines (Huh7, Hep3B, SNU449, and SNU475).
Fig. 5 shows dose response curves in various cancer cells.
Fig. 6 shows dose response curves in hepatoma cell lines (Huh7 and Hep3B).
Fig. 7 shows dose response curves in non-small cell lung cancer cell lines (NCI-H460 and NCI-H1299).
Fig. 8 shows dose response curves in pancreatic cancer cell lines (PANC-1 and Mia-Paca-2).
Fig. 9 shows dose response curves in colon cancer cell lines (HT29 and HCT116).
Fig. 10 shows a dose response curve in a gastric adenocarcinoma cell line (AGS).
Fig. 11 shows a dose response curve in a malignant melanoma cell line (A375).
Fig. 12 shows a dose response curve in an adenocarcinoma cell line (MDA-MB-231).
Figs. 13 and 14 show the results of the CRBN affinity test of the compounds in the examples of the present invention.

### Best Mode for Carrying out the Invention

The terms in this specification are used to appropriately express preferred embodiments of the present invention, and the definitions of these terms should be made based on the contents throughout this specification. Throughout the specification, when a part "includes" a certain component, this does not mean that it excludes other components, but rather that it may further include other components, unless otherwise stated.

All technical terms used in the present invention, unless otherwise defined, have the same meaning as commonly understood by an average person skilled in the art in the relevant field of the present invention. Although preferred methods and samples are described in this specification, similar or equivalent methods and samples are also included in the scope of the present invention.

In an aspect of the present invention, the present invention relates to a compound represented by the following Formula 1, or to an enantiomeric compound thereof: 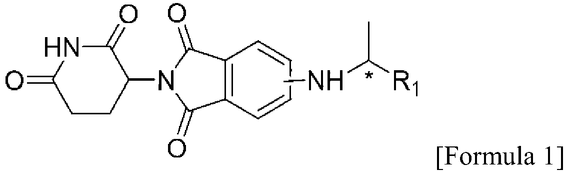
where in Formula 1 above,
the R₁ is alkyl, saturated or unsaturated cycloalkyl, aromatic or non-aromatic heterocyclyl, aryl, or -alkyl-C(=O)-O-alkyl;
the alkyl is unsubstituted or substituted with one or more alkoxy groups or hydroxy groups; and
the heterocyclyl or aryl is unsubstituted or substituted with one or more alkyl groups, hydroxy groups, halogens, alkoxy groups, -(C=O)-O-alkyl, -CF₃, -OCF₃, -S-alkyl, -S(O₂)-alkyl, or -S(O₂)NH₂.

As used herein, the term "alkyl" refers to a linear or branched saturated hydrocarbon residue unless otherwise specified. In one aspect of the present invention, the alkyl may be C₁₋₇ alkyl, which means an alkyl having a backbone of 1 to 7 carbons. Specifically, the C₁₋₇ alkyl may be methyl, ethyl, n-propyl, i-propyl, n-butyl, i-butyl, t-butyl, n-pentyl, i-pentyl, t-pentyl, sec-pentyl, neopentyl, hexyl, heptyl, *etc.*

As used herein, the term "cycloalkyl" refers to a cyclic hydrocarbon residue, including saturated or unsaturated cycloalkyl. In one aspect of the present invention, the cycloalkyl may have a cyclic backbone consisting of 3 to 7 carbons. Specifically, cycloalkyl may be cyclopropyl, cyclobutyl, cyclopentyl, cyclohexyl, cycloheptyl, cyclopropenyl, cyclobutenyl, cyclopentenyl, cyclohexenyl, or cycloheptenyl.

As used herein, the term "aromatic or non-aromatic heterocyclyl" is a ring system including one or more aromatic or non-aromatic rings, in which one or more atoms are heteroatoms independently selected from O, S, N, and P, the remaining ring atoms are carbon atoms, and one or more double bonds are included. In one aspect of the present invention, the heterocyclyl may be an aromatic or non-aromatic heterocyclyl of a 5- to 12-membered ring including a heteroatom selected from the group consisting of N, O, and S. For example, the heterocyclyl may be benzofuran, dihydrobenzofuran, pyridine, pyrrolidine, piperidine, or pyrimidine, but is not limited thereto.

As used herein, the term "saturated" compound refers to a compound in which all carbon-carbon bonds consist of single bonds, while the term "unsaturated" compound refers to a compound that includes one or more double bonds or triple bonds.

As used herein, the term "aryl" refers to a monocyclic aromatic group and/or polycyclic aromatic group including one or more aromatic hydrocarbon rings. In one aspect of the present invention, the aryl may have a cyclic backbone consisting of 6 to 12 carbons. Examples of the aryl group include phenyl, naphthyl, and azulenyl, but are not limited thereto.

As used herein, the term "halogen" refers to F, Cl, Br, or I, unless otherwise specified.

As used herein, the term "alkoxy" refers to a linear or branched alkyl-oxy moiety, unless otherwise specified. In one aspect of the present invention, the alkoxy may be a "C₁₋₆ alkoxy", which means an alkyl-oxy having a backbone consisting of 1 to 6 carbons. Specifically, the C₁₋₆ alkoxy may be methoxy, ethoxy, n-propoxy, i-propoxy, n-butoxy, i-butoxy, t- butoxy, n-pentoxy, i-penoxy, t-pentoxy, sec-pentoxy, neopentoxy, hexyloxy, *etc.*

As used herein, the term "substitution" means that a hydrogen atom attached to a carbon atom in a structure is substituted with another substituent, in which the position of substitution is not limited to the position where the hydrogen atom is substituted, that is, it is not limited to any position where the substituent may be substituted, and in the case where the substitution occurs at two or more positions, the two or more substituents may be the same or different from one another.

Additionally, the compound of the present invention may have a chiral carbon center, and thus it may exist in the form of an R or S isomer, a racemic compound, an individual enantiomer or mixture, or an individual diastereomer or mixture, and all these stereoisomers and mixtures thereof may fall within the category of the present invention.

In an embodiment of the present invention, R₁ of Formula 1 above may be C₁₋₇ alkyl, C₃₋₇ cycloalkyl, an aromatic or non-aromatic heterocyclyl having a 5- to 12 membered ring including a heteroatom selected from the group consisting of N, O, and S, or C₆₋₁₂ aryl.

In an embodiment of the present invention, R₁ of Formula 1 above may be substituted or unsubstituted C₁₋₅ alkyl, substituted or unsubstituted C₃₋₅ cycloalkyl, substituted or unsubstituted dihydrobenzofuran or pyridine, substituted or unsubstituted phenyl, or C₁₋₅ alkyl-(C=O)-O-alkyl.

In an embodiment of the present invention, R₁ of Formula 1 above may be C₁₋₃ alkyl substituted with a hydroxy group; unsubstituted C₃₋₅ alkyl; or phenyl substituted with a methoxy group.

In an embodiment of the present invention, the compound represented by Formula 1 above may be any one selected from the group consisting of the following:
1) methyl (3R)-3-((2-(2,6-dioxopiperidin-3-yl)-1,3-dioxoisoindolin-4-yl)amino)butanoate;
2) methyl (3S)-3-((2-(2,6-dioxopiperidin-3-yl)-1,3-dioxoisoindolin-4-yl)amino)butanoate;
3) 2-(2,6-dioxopiperidin-3-yl)-4-(((S)-1-hydroxypropan-2-yl)amino)isoindoline-1,3-dione;
4) 2-(2,6-dioxopiperidin-3-yl)-4-(((R)-1-hydroxypropan-2-yl)amino)isoindoline-1,3-dione;
5) 2-(2,6-dioxopiperidin-3-yl)-4-(((S)-pentan-2-yl)amino)isoindoline-1,3-dione;
6) 2-(2,6-dioxopiperidin-3-yl)-4-((3-methylbutan-2-yl)amino)isoindoline-1,3-dione;
7) 4-((3,3-dimethylbutan-2-yl)amino)-2-(2,6-dioxopiperidin-3-yl)isoindoline-1,3-dione;
8) 2-(2,6-dioxopiperidin-3-yl)-4-(((S)-1-methoxypropan-2-yl)amino)isoindoline-1,3-dione;
9) 2-(2,6-dioxopiperidin-3-yl)-4-(((R)-1-(4-fluorophenyl)ethyl)amino)isoindoline-1,3-dione;
10) 2-(2,6-dioxopiperidin-3-yl)-4-(((S)-1-(4-fluorophenyl)ethyl)amino)isoindoline-1,3-dione;
11) 2-(2,6-dioxopiperidin-3-yl)-4-(((S)-1-(4-methoxyphenyl)ethyl)amino)isoindoline-1,3-dione;
12) 2-(2,6-dioxopiperidin-3-yl)-4-(((R)-1-(4-methoxyphenyl)ethyl)amino)isoindoline-1,3-dione;
13) 2-(2,6-dioxopiperidin-3-yl)-4-(((R)-1-(4-(trifluoromethoxy)phenyl)ethyl)amino)isoindoline-1,3-dione;
14) 2-(2,6-dioxopiperidin-3-yl)-4-(((S)-1-(4-(trifluoromethoxy)phenyl)ethyl)amino)isoindoline-1,3-dione;
15) 2-(2,6-dioxopiperidin-3-yl)-4-(((R)-1-(2-fluorophenyl)ethyl)amino)isoindoline-1,3-dione;
16) 2-(2,6-dioxopiperidin-3-yl)-4-(((S)-1-(2-fluorophenyl)ethyl)amino)isoindoline-1,3-dione;
17) 2-(2,6-dioxopiperidin-3-yl)-4-(((R)-1-(3-methoxyphenyl)ethyl)amino)isoindoline-1,3-dione;
18) 2-(2,6-dioxopiperidin-3-yl)-4-(((S)-1-(3-methoxyphenyl)ethyl)amino)isoindoline-1,3-dione;
19) 2-(2,6-dioxopiperidin-3-yl)-4-(((R)-1-(2-(trifluoromethyl)phenyl)ethyl)amino)isoindoline-1,3-dione;
20) 4-((1R)-1-((2-(2,6-dioxopiperidin-3-yl)-1,3-dioxoisoindolin-4-yl)amino)ethyl)benzenesulfonamide;
21) methyl 4-((1R)-1-((2-(2,6-dioxopiperidin-3-yl)-1,3-dioxoisoindolin-4-yl)amino)ethyl)benzoate;
22) methyl 4-((1S)-1-((2-(2,6-dioxopiperidin-3-yl)-1,3-dioxoisoindolin-4-yl)amino)ethyl)benzoate;
23) 2-(2,6-dioxopiperidin-3-yl)-4-(((R)-1-(p-tolyl)ethyl)amino)isoindoline-1,3-dione;
24) 2-(2,6-dioxopiperidin-3-yl)-4-(((S)-1-(p-tolyl)ethyl)amino)isoindoline-1,3-dione;
25) 4-(((R)-1-(4-chlorophenyl)ethyl)amino)-2-(2,6-dioxopiperidin-3-yl)isoindoline-1,3-dione;
26) 4-(((S)-1-(4-chlorophenyl)ethyl)amino)-2-(2,6-dioxopiperidin-3-yl)isoindoline-1,3-dione;
27) 2-(2,6-dioxopiperidin-3-yl)-4-(((R)-1-(2-fluoro-4-methoxyphenyl)ethyl)amino)isoindoline-1,3-dione;
28) 2-(2,6-dioxopiperidin-3-yl)-4-(((S)-1-(2-fluoro-4-methoxyphenyl)ethyl)amino)isoindoline-1,3-dione;
29) 2-(2,6-dioxopiperidin-3-yl)-4-(((R)-1-(4-fluoro-3-methoxyphenyl)ethyl)amino)isoindoline-1,3-dione;
30) 2-(2,6-dioxopiperidin-3-yl)-4-(((S)-1-(4-fluoro-3-methoxyphenyl)ethyl)amino)isoindoline-1,3-dione;
31) 2-(2,6-dioxopiperidin-3-yl)-4-(((R)-1-(4-(trifluoromethyl)phenyl)ethyl)amino)isoindoline-1,3-dione;
32) 2-(2,6-dioxopiperidin-3-yl)-4-(((S)-1-(4-(trifluoromethyl)phenyl)ethyl)amino)isoindoline-1,3-dione;
33) 2-(2,6-dioxopiperidin-3-yl)-4-(((R)-1-(4-(methylthio)phenyl)ethyl)amino)isoindoline-1,3-dione;
34) 2-(2,6-dioxopiperidin-3-yl)-4-(((S)-1-(4-(methylthio)phenyl)ethyl)amino)isoindoline-1,3-dione;
35) 2-(2,6-dioxopiperidin-3-yl)-4-(((S)-1-(4-ethoxyphenyl)ethyl)amino)isoindoline-1,3-dione;
36) 2-(2,6-dioxopiperidin-3-yl)-4-(((R)-1-(3-fluorophenyl)ethyl)amino)isoindoline-1,3-dione;
37) 2-(2,6-dioxopiperidin-3-yl)-4-(((S)-1-(3-fluorophenyl)ethyl)amino)isoindoline-1,3-dione;
38) 2-(2,6-dioxopiperidin-3-yl)-4-(((R)-1-(3-fluoro-4-methoxyphenyl)ethyl)amino)isoindoline-1,3-dione;
39) 4-(((R)-1-(3,4-difluorophenyl)ethyl)amino)-2-(2,6-dioxopiperidin-3-yl)isoindoline-1,3-dione;
40) 4-(((S)-1-(3,4-difluorophenyl)ethyl)amino)-2-(2,6-dioxopiperidin-3-yl)isoindoline-1,3-dione;
41) 4-(((R)-1-(3,5-difluorophenyl)ethyl)amino)-2-(2,6-dioxopiperidin-3-yl)isoindoline-1,3-dione;
42) 4-(((S)-1-(3,5-difluorophenyl)ethyl)amino)-2-(2,6-dioxopiperidin-3-yl)isoindoline-1,3-dione;
43) 2-(2,6-dioxopiperidin-3-yl)-4-(((R)-1-(6-methoxypyridin-3-yl)ethyl)amino)isoindoline-1,3-dione;
44) 2-(2,6-dioxopiperidin-3-yl)-4-(((S)-1-(6-methoxypyridin-3-yl)ethyl)amino)isoindoline-1,3-dione;
45) 4-(((R)-1-(3,5-dimethoxyphenyl)ethyl)amino)-2-(2,6-dioxopiperidin-3-yl)isoindoline-1,3-dione;
46) 2-(2,6-dioxopiperidin-3-yl)-4-(((S)-1-(3-fluoro-4-methoxyphenyl)ethyl)amino)isoindoline-1,3-dione;
47) 2-(2,6-dioxopiperidin-3-yl)-4-(((R)-1-(5-methoxypyridin-2-yl)ethyl)amino)isoindoline-1,3-dione;
48) 2-(2,6-dioxopiperidin-3-yl)-4-(((S)-1-(5-methoxypyridin-2-yl)ethyl)amino)isoindoline-1,3-dione;
49) 2-(2,6-dioxopiperidin-3-yl)-4-(((R)-1-(4-(methylsulfonyl)phenyl)ethyl)amino)isoindoline-1,3-dione;
50) 2-(2,6-dioxopiperidin-3-yl)-4-(((S)-1-(4-(methylsulfonyl)phenyl)ethyl)amino)isoindoline-1,3-dione;
51) 4-(((R)-1-(2,3-dihydrobenzofuran-5-yl)ethyl)amino)-2-(2,6-dioxopiperidin-3-yl)isoindoline-1,3-dione; and
52) 4-(((S)-1-cyclopropylethyl)amino)-2-(2,6-dioxopiperidin-3-yl)isoindoline-1,3-dione.

In an embodiment of the present invention, the compound represented by Formula 1 above may be 2-(2,6-dioxopiperidin-3-yl)-4-(((R)-1-hydroxypropan-2-yl)amino)isoindoline-1,3-dione; 2-(2,6-dioxopiperidin-3-yl)-4-(((S)-pentan-2-yl)amino)isoindoline-1,3-dione; or 2-(2,6-dioxopiperidin-3-yl)-4-(((R)-1-(4-methoxyphenyl)ethyl)amino)isoindoline-1,3-dione.

In an embodiment of the present invention, the compound represented by Formula 1 above may function as a molecular glue. Specifically, the compound represented by Formula 1 above may be a molecular glue substance that binds to the Cereblon (or CRBN) protein and exhibits an anticancer effect through the mechanism of an immunomodulatory imide drug (IMiD).

In an embodiment of the present invention, the protein utilized as an endogenous target protein of immunomodulators may be a CRBN protein. CRBN proteins form an E3 ubiquitin ligase complex with damaged DNA binding protein 1 (DDB1), cullin-4A (CUL4A), and a regulator of cullin 1 (ROC1), and this complex ubiquitinates numerous other proteins. CRBN proteins are substrate receptors for CRL4, which is an E3 ubiquitin ligase, and CRL4/CRBN may regulate the intracellular homeostasis of several important intracellular proteins through ubiquitin-dependent degradation of these proteins; additionally, the CRBN proteins may directly bind to AMPK, which may cause a wide range of metabolic diseases such as cancer. Moreover, the CRBN proteins may also play a negative regulatory role in the activation of CD4+ T cells. CRBN deficiency increases CD4+ T cell activation and enhances IL-2 secretion, thereby inducing the differentiation of CD4+ T cells into Th17 cells. Enhanced T cell activation may sometimes lead to T cell-mediated autoimmune diseases, such as autoimmune encephalomyelitis and delayed-type hypersensitivity.

In still another aspect, the present invention relates to a pharmaceutical composition for preventing or treating cancer, which includes a compound represented by Formula 1 above, or an enantiomeric compound thereof as an active ingredient, in which the cancer may be any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.

As used herein, the term "treatment" refers to any act by which the symptoms of a disease are improved or beneficially changed by administering a composition according to the present invention; and the term "prevention" refers to any act of inhibiting or delaying a disease by administering a composition according to the present invention; and the term "improvement" refers to any act of improving a bad condition of a disease by administering or allowing the ingestion of the composition of the present invention to a subject.

The disease targeted for "treatment," "prevention", or "improvement" of the present invention may be cancer. Cancer refers to a physiological condition in animals that is typically characterized by abnormal or uncontrolled cell growth. Cancer and cancer pathology may be associated with, for example, metastasis, interference with normally functioning surrounding cells, release of cytokines or other secreted products at abnormal levels, inhibition or enhancement of inflammatory or immunological responses, neoplasia, premalignancy, or malignancy.

In an embodiment of the present invention, the cancer may be a recurrent or incurable cancer.

In an embodiment of the present invention, the cancer may be a drug-resistant cancer.

The pharmaceutical composition of the present invention may be administered in a therapeutically effective amount of the active ingredient. The therapeutically effective amount refers to the amount of drug administered that exhibits an effective effect on the prevention or treatment of cancer. The appropriate total daily dose may be determined by a practicing physician within the scope of sound medical judgment. The specific therapeutically effective dose for a particular patient may be applied differently depending on various factors including the type and degree of the response to be achieved, the type and amount of drugs to be co-administered, the specific composition including whether other preparations are used in some cases, the patient's age, weight, general health condition, sex and diet, the time period of administration, the route of administration, the duration of treatment, and similar factors well known in the medical field.

The pharmaceutical composition of the present invention may be administered to a subject or individual by any route of administration, for example, it may be administered by intraperitoneal administration, intravenous administration, intramuscular administration, subcutaneous administration, intradermal injection, oral administration, topical administration, intranasal injection, intrapulmonary injection, or rectal administration route. Preferably, the pharmaceutical composition of the present invention may be administered orally or intravenously.

In an embodiment of the present invention, the active ingredient of the pharmaceutical composition may be any one selected from the group consisting of the following:
1) methyl (3R)-3-((2-(2,6-dioxopiperidin-3-yl)-1,3-dioxoisoindolin-4-yl)amino)butanoate;
2) methyl (3S)-3-((2-(2,6-dioxopiperidin-3-yl)-1,3-dioxoisoindolin-4-yl)amino)butanoate;
3) 2-(2,6-dioxopiperidin-3-yl)-4-(((S)-1-hydroxypropan-2-yl)amino)isoindoline-1,3-dione;
4) 2-(2,6-dioxopiperidin-3-yl)-4-(((R)-1-hydroxypropan-2-yl)amino)isoindoline-1,3-dione;
5) 2-(2,6-dioxopiperidin-3-yl)-4-(((S)-pentan-2-yl)amino)isoindoline-1,3-dione;
6) 2-(2,6-dioxopiperidin-3-yl)-4-((3-methylbutan-2-yl)amino)isoindoline-1,3-dione;
7) 4-((3,3-dimethylbutan-2-yl)amino)-2-(2,6-dioxopiperidin-3-yl)isoindoline-1,3-dione;
8) 2-(2,6-dioxopiperidin-3-yl)-4-(((S)-1-methoxypropan-2-yl)amino)isoindoline-1,3-dione;
9) 2-(2,6-dioxopiperidin-3-yl)-4-(((R)-1-(4-fluorophenyl)ethyl)amino)isoindoline-1,3-dione;
10) 2-(2,6-dioxopiperidin-3-yl)-4-(((S)-1-(4-fluorophenyl)ethyl)amino)isoindoline-1,3-dione;
11) 2-(2,6-dioxopiperidin-3-yl)-4-(((S)-1-(4-methoxyphenyl)ethyl)amino)isoindoline-1,3-dione;
12) 2-(2,6-dioxopiperidin-3-yl)-4-(((R)-1-(4-methoxyphenyl)ethyl)amino)isoindoline-1,3-dione;
13) 2-(2,6-dioxopiperidin-3-yl)-4-(((R)-1-(4-(trifluoromethoxy)phenyl)ethyl)amino)isoindoline-1,3-dione;
14) 2-(2,6-dioxopiperidin-3-yl)-4-(((S)-1-(4-(trifluoromethoxy)phenyl)ethyl)amino)isoindoline-1,3-dione;
15) 2-(2,6-dioxopiperidin-3-yl)-4-(((R)-1-(2-fluorophenyl)ethyl)amino)isoindoline-1,3-dione;
16) 2-(2,6-dioxopiperidin-3-yl)-4-(((S)-1-(2-fluorophenyl)ethyl)amino)isoindoline-1,3-dione;
17) 2-(2,6-dioxopiperidin-3-yl)-4-(((R)-1-(3-methoxyphenyl)ethyl)amino)isoindoline-1,3-dione;
18) 2-(2,6-dioxopiperidin-3-yl)-4-(((S)-1-(3-methoxyphenyl)ethyl)amino)isoindoline-1,3-dione;
19) 2-(2,6-dioxopiperidin-3-yl)-4-(((R)-1-(2-(trifluoromethyl)phenyl)ethyl)amino)isoindoline-1,3-dione;
20) 4-((1R)-1-((2-(2,6-dioxopiperidin-3-yl)-1,3-dioxoisoindolin-4-yl)amino)ethyl)benzenesulfonamide;
21) methyl 4-((1R)-1-((2-(2,6-dioxopiperidin-3-yl)-1,3-dioxoisoindolin-4-yl)amino)ethyl)benzoate;
22) methyl 4-((1S)-1-((2-(2,6-dioxopiperidin-3-yl)-1,3-dioxoisoindolin-4-yl)amino)ethyl)benzoate;
23) 2-(2,6-dioxopiperidin-3-yl)-4-(((R)-1-(p-tolyl)ethyl)amino)isoindoline-1,3-dione;
24) 2-(2,6-dioxopiperidin-3-yl)-4-(((S)-1-(p-tolyl)ethyl)amino)isoindoline-1,3-dione;
25) 4-(((R)-1-(4-chlorophenyl)ethyl)amino)-2-(2,6-dioxopiperidin-3-yl)isoindoline-1,3-dione;
26) 4-(((S)-1-(4-chlorophenyl)ethyl)amino)-2-(2,6-dioxopiperidin-3-yl)isoindoline-1,3-dione;
27) 2-(2,6-dioxopiperidin-3-yl)-4-(((R)-1-(2-fluoro-4-methoxyphenyl)ethyl)amino)isoindoline-1,3-dione;
28) 2-(2,6-dioxopiperidin-3-yl)-4-(((S)-1-(2-fluoro-4-methoxyphenyl)ethyl)amino)isoindoline-1,3-dione;
29) 2-(2,6-dioxopiperidin-3-yl)-4-(((R)-1-(4-fluoro-3-methoxyphenyl)ethyl)amino)isoindoline-1,3-dione;
30) 2-(2,6-dioxopiperidin-3-yl)-4-(((S)-1-(4-fluoro-3-methoxyphenyl)ethyl)amino)isoindoline-1,3-dione;
31) 2-(2,6-dioxopiperidin-3-yl)-4-(((R)-1-(4-(trifluoromethyl)phenyl)ethyl)amino)isoindoline-1,3-dione;
32) 2-(2,6-dioxopiperidin-3-yl)-4-(((S)-1-(4-(trifluoromethyl)phenyl)ethyl)amino)isoindoline-1,3-dione;
33) 2-(2,6-dioxopiperidin-3-yl)-4-(((R)-1-(4-(methylthio)phenyl)ethyl)amino)isoindoline-1,3-dione;
34) 2-(2,6-dioxopiperidin-3-yl)-4-(((S)-1-(4-(methylthio)phenyl)ethyl)amino)isoindoline-1,3-dione;
35) 2-(2,6-dioxopiperidin-3-yl)-4-(((S)-1-(4-ethoxyphenyl)ethyl)amino)isoindoline-1,3-dione;
36) 2-(2,6-dioxopiperidin-3-yl)-4-(((R)-1-(3-fluorophenyl)ethyl)amino)isoindoline-1,3-dione;
37) 2-(2,6-dioxopiperidin-3-yl)-4-(((S)-1-(3-fluorophenyl)ethyl)amino)isoindoline-1,3-dione;
38) 2-(2,6-dioxopiperidin-3-yl)-4-(((R)-1-(3-fluoro-4-methoxyphenyl)ethyl)amino)isoindoline-1,3-dione;
39) 4-(((R)-1-(3,4-difluorophenyl)ethyl)amino)-2-(2,6-dioxopiperidin-3-yl)isoindoline-1,3-dione;
40) 4-(((S)-1-(3,4-difluorophenyl)ethyl)amino)-2-(2,6-dioxopiperidin-3-yl)isoindoline-1,3-dione;
41) 4-(((R)-1-(3,5-difluorophenyl)ethyl)amino)-2-(2,6-dioxopiperidin-3-yl)isoindoline-1,3-dione;
42) 4-(((S)-1-(3,5-difluorophenyl)ethyl)amino)-2-(2,6-dioxopiperidin-3-yl)isoindoline-1,3-dione;
43) 2-(2,6-dioxopiperidin-3-yl)-4-(((R)-1-(6-methoxypyridin-3-yl)ethyl)amino)isoindoline-1,3-dione;
44) 2-(2,6-dioxopiperidin-3-yl)-4-(((S)-1-(6-methoxypyridin-3-yl)ethyl)amino)isoindoline-1,3-dione;
45) 4-(((R)-1-(3,5-dimethoxyphenyl)ethyl)amino)-2-(2,6-dioxopiperidin-3-yl)isoindoline-1,3-dione;
46) 2-(2,6-dioxopiperidin-3-yl)-4-(((S)-1-(3-fluoro-4-methoxyphenyl)ethyl)amino)isoindoline-1,3-dione;
47) 2-(2,6-dioxopiperidin-3-yl)-4-(((R)-1-(5-methoxypyridin-2-yl)ethyl)amino)isoindoline-1,3-dione;
48) 2-(2,6-dioxopiperidin-3-yl)-4-(((S)-1-(5-methoxypyridin-2-yl)ethyl)amino)isoindoline-1,3-dione;
49) 2-(2,6-dioxopiperidin-3-yl)-4-(((R)-1-(4-(methylsulfonyl)phenyl)ethyl)amino)isoindoline-1,3-dione;
50) 2-(2,6-dioxopiperidin-3-yl)-4-(((S)-1-(4-(methylsulfonyl)phenyl)ethyl)amino)isoindoline-1,3-dione;
51) 4-(((R)-1-(2,3-dihydrobenzofuran-5-yl)ethyl)amino)-2-(2,6-dioxopiperidin-3-yl)isoindoline-1,3-dione; and
52) 4-(((S)-1-cyclopropylethyl)amino)-2-(2,6-dioxopiperidin-3-yl)isoindoline-1,3-dione.

In an embodiment of the present invention, the active ingredient may be 2-(2,6-dioxopiperidin-3-yl)-4-(((R)-1-hydroxypropan-2-yl)amino)isoindoline-1,3-dione; 2-(2,6-dioxopiperidin-3-yl)-4-(((S)-pentan-2-yl)amino)isoindoline-1,3-dione; or 2-(2,6-dioxopiperidin-3-yl)-4-(((R)-1-(4-methoxyphenyl)ethyl)amino)isoindoline-1,3-dione.

In still another aspect, the present invention relates to a method for preventing or treating cancer in a subject, which includes administering, to a subject, a compound represented by Formula 1 above, or an enantiomeric compound thereof, wherein the cancer is any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.

As used herein, the term "subject" is used synonymously with "individual", wherein the subject may be a mammal, for example a human, a mouse, a cow, a horse, a pig, a dog, a sheep, a goat or a cat.

Any content that overlaps with the pharmaceutical composition of the present invention described above is omitted.

In still another aspect, the present invention relates to a use of a compound represented by Formula 1 above, or an enantiomeric compound thereof, for the preparation of a medicament for preventing or treating cancer, wherein the cancer is any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.

Any content that overlaps with the pharmaceutical composition of the present invention described above is omitted.

In still another aspect, the present invention relates to a use of a compound represented by Formula 1 above, or an enantiomeric compound thereof, wherein the cancer is any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.

Any content that overlaps with the pharmaceutical composition of the present invention described above is omitted.

The matters mentioned in all of the compositions, treatment methods, and uses of the present invention are equally applicable unless they are contradictory to one another.

Hereinafter, preferred embodiments are presented to help understand the present invention, but the following embodiments are only an illustration of the present invention, and it is obvious to those skilled in the art that various changes and modifications are possible within the category and technical scope of the present invention, and it is also natural that such changes and modifications fall within the scope of the appended claims.

### [Mode for the Invention]

### Example

The novel compound of the present invention can be prepared by the following Reaction Scheme 1, but is not limited thereto.

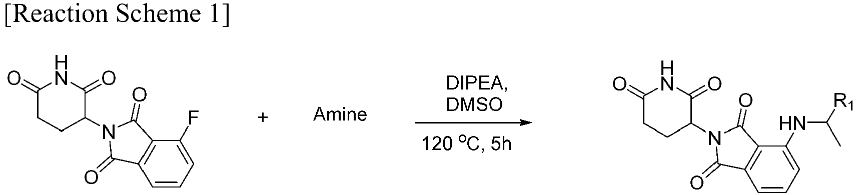

2-(2,6-dioxopiperidin-3-yl)-4-fluoroisoindoline-1,3-dione (Starting Material 1, 1 equivalent, purchased from EOS, China), amine (2 equivalents, purchased from Aldrich), and N,N-diisopropylethylamine (DIPEA, 2 equivalents, purchased from Aldrich) were added into a solvent of dimethyl sulfoxide (DMSO, purchased from Aldrich, 8 times the volume of Starting Material 1) at room temperature. Then, the temperature increased to 120°C, and the mixture was stirred for 5 to 8 hours until less than 5% of the starting material remained, as analyzed by HPLC. After the completion of the reaction, the resultant was cooled slowly to room temperature, purified water (twice the amount compared to DMSO) was slowly added thereto, and the resulting solid was filtered under reduced pressure. The obtained solid was recrystallized with isopropyl alcohol to obtain a solid with a purity of 98% or more.

As shown in Table 1 below, the compounds for Examples were obtained by using the method above in preparation while changing the type of amine in Reaction Scheme 1 above.

**[Table 1]**

| No. | Amine | Compound | Yield |
|---|---|---|---|
| 1 | 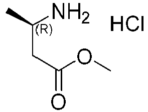 methyl (R)-3-aminobutanoate hydrochloride | 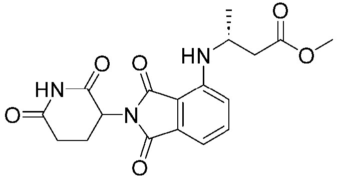 methyl (3R)-3-((2-(2,6-dioxopiperidin-3-yl)-1,3-dioxoisoindolin-4-yl)amino)butanoate | 45% |
| 2 | 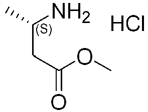 methyl (S)-3-aminobutanoate hydrochloride | 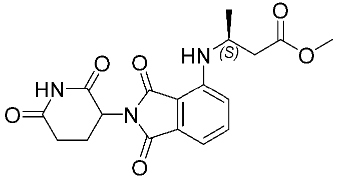 methyl (3S)-3-((2-(2,6-dioxopiperidin-3-yl)-1,3-dioxoisoindolin-4-yl)amino)butanoate | 27% |
| 3 | 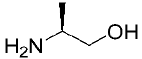 (S)-2-aminopropan-1-ol | 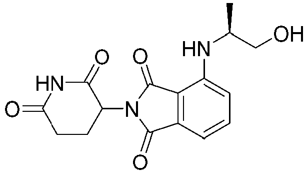 2-(2,6-dioxopiperidin-3-yl)-4-(((S)-1-hydroxypropan-2-yl)amino)isoindoline-1,3-dione | 26% |
| 4 (AST-DT-220) | 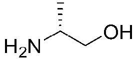 (R)-2-aminopropan-1-ol | 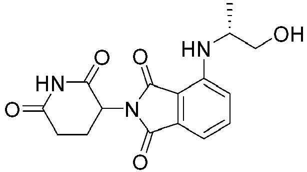 2-(2,6-dioxopiperidin-3-yl)-4-(((R)-1-hydroxypropan-2-yl)amino)isoindoline-1,3-dione | 29% |
| 5 (AST-DT-135) | 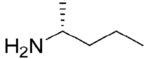 (S)-pentan-2-amine | 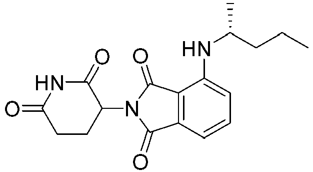 2-(2,6-dioxopiperidin-3-yl)-4-(((S)-pentan-2-yl)amino)isoindoline-1,3-dione | 34% |
| 6 | 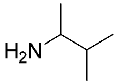 3-methylbutan-2-amine | 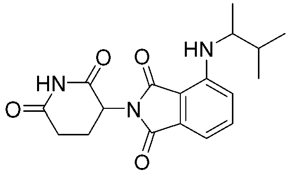 2-(2,6-dioxopiperidin-3-yl)-4-((3-methylbutan-2-yl)amino)isoindoline-1,3-dione | 33% |
| 7 | 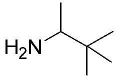 3,3-dimethylbutan-2-amine | 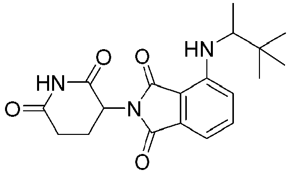 4-((3,3-dimethylbutan-2-yl)amino)-2-(2,6-dioxopiperidin-3-yl)isoindoline-1,3-dione | 48% |
| 8 | 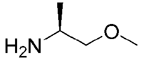 (S)-1-methoxypropan-2-amine | 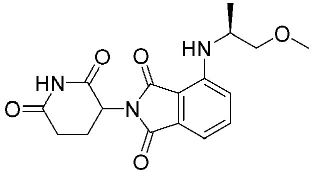 2-(2,6-dioxopiperidin-3-yl)-4-(((S)-1-methoxypropan-2-yl)amino)isoindoline-1,3-dione | 36% |
| 9 | 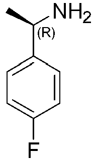 (R)-1-(4-fluorophenyl)ethan-1-amine | 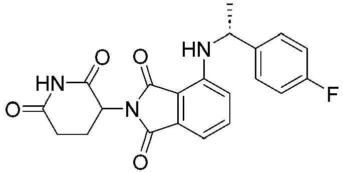 2-(2,6-dioxopiperidin-3-yl)-4-(((R)-1-(4-fluorophenyl)ethyl)amino)isoindoline-1,3-dione | 68% |
| 10 | 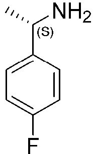 (S)-1-(4-fluorophenyl)ethan-1-amine | 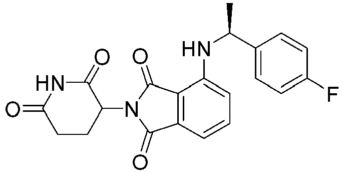 2-(2,6-dioxopiperidin-3-yl)-4-(((S)-1-(4-fluorophenyl)ethyl)amino)isoindoline-1,3-dione | 56% |
| 11 | 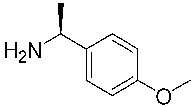 (S)-1-(4-methoxyphenyl)ethan-1-amine | 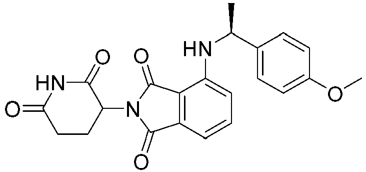 2-(2,6-dioxopiperidin-3-yl)-4-(((S)-1-(4-methoxyphenyl)ethyl)amino)isoindoline -1,3-dione | 29% |
| 12 (AST-DT-218) | 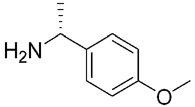 (R)-1-(4-methoxyphenyl)ethan-1-amine | 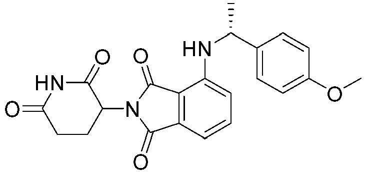 2-(2,6-dioxopiperidin-3-yl)-4-(((R)-1-(4-methoxyphenyl)ethyl)amino)isoindoline-1,3-dione | 68% |
| 13 | 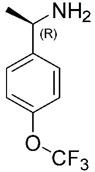 (R)-1-(4-(trifluoromethoxy)phen yl)ethan-1-amine | 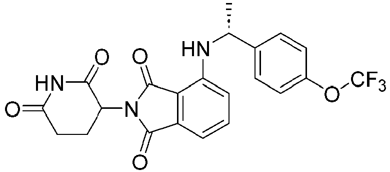 2-(2,6-dioxopiperidin-3-yl)-4-(((R)-1-(4-(trifluoromethoxy)phenyl)ethyl)amino) isoindoline-1,3-dione | 47% |
| 14 | 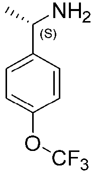 (S)-1-(4-(trifluoromethoxy)phen yl)ethan-1-amine | 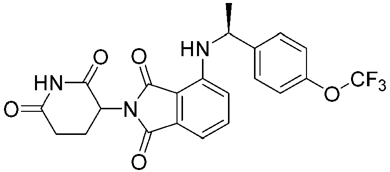 2-(2,6-dioxopiperidin-3-yl)-4-(((S)-1-(4-(trifluoromethoxy)phenyl)ethyl)amino) isoindoline-1,3-dione | 42% |
| 15 | 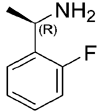 (R)-1-(2-fluorophenyl)ethan-1-amine | 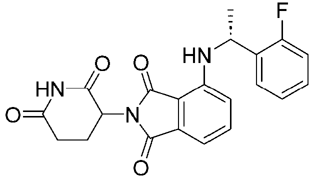 2-(2,6-dioxopiperidin-3-yl)-4-(((R)-1-(2-fluorophenyl)ethyl)amino)isoindoline-1,3-dione | 51% |
| 16 | 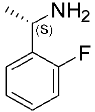 (S)-1-(2-fluorophenyl)ethan-1-amine | 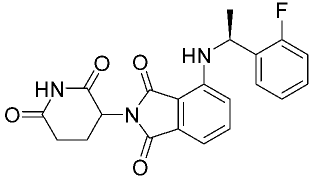 2-(2,6-dioxopiperidin-3-yl)-4-(((S)-1-(2-fluorophenyl)ethyl)amino)isoindoline-1,3-dione | 44% |
| 17 | 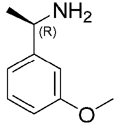 (R)-1-(3-methoxyphenyl)ethan-1-amine | 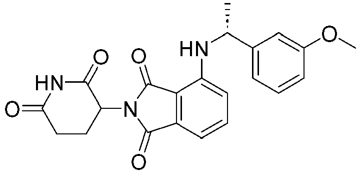 2-(2,6-dioxopiperidin-3-yl)-4-(((R)-1-(3-methoxyphenyl)ethyl)amino)isoindoline -1,3-dione | 46% |
| 18 | 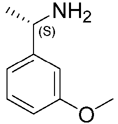 (S)-1-(3-methoxyphenyl)ethan-1-amine | 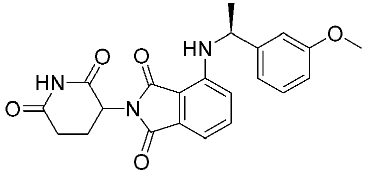 2-(2,6-dioxopiperidin-3-yl)-4-(((S)-1-(3-methoxyphenyl)ethyl)amino)isoindoline -1,3-dione | 65% |
| 19 | 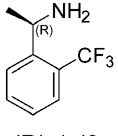 (R)-1-(2-(trifluoromethyl)phenyl) ethan-1-amine | 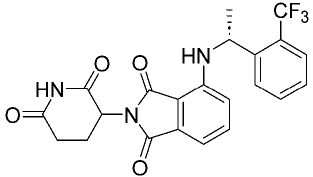 2-(2,6-dioxopiperidin-3-yl)-4-(((R)-1-(2-(trifluoromethyl)phenyl)ethyl)amino)is oindoline-1,3-dione | 56% |
| 20 | 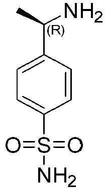 (R)-4-(1-aminoethyl)benzenesul fonamide | 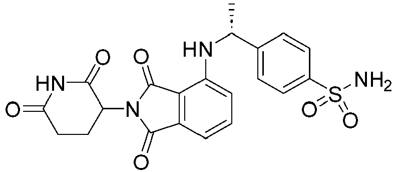 4-((1R)-1-((2-(2,6-dioxopiperidin-3-yl)-1,3-dioxoisoindolin-4-yl)amino)ethyl)benzenesulfonamide | 67% |
| 21 | 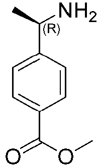 methyl (R)-4-(1-aminoethyl)benzoate | 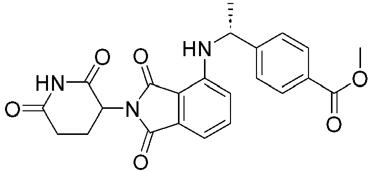 methyl 4-((1R)-1-((2-(2,6-dioxopiperidin-3-yl)-1,3-dioxoisoindolin-4-yl)amino)ethyl)benzoate | 45% |
| 22 | 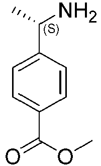 methyl (S)-4-(1-aminoethyl)benzoate | 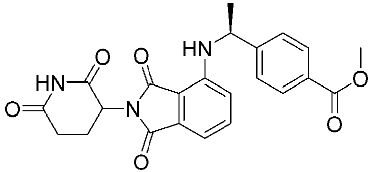 methyl 4-((1S)-1-((2-(2,6-dioxopiperidin-3-yl)-1,3-dioxoisoindolin-4-yl)amino)ethyl)benzoate | 40% |
| 23 | 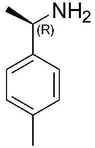 (R)-1-(p-tolyl)ethan-1-amine | 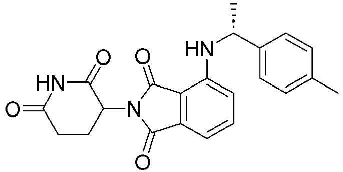 2-(2,6-dioxopiperidin-3-yl)-4-(((R)-1-(p-tolyl)ethyl)amino)isoindoline-1,3-dione | 58% |
| 24 | 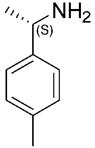 (S)-1-(p-tolyl)ethan-1-amine | 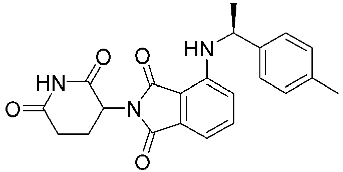 2-(2,6-dioxopiperidin-3-yl)-4-(((S)-1-(p-tolyl)ethyl)amino)isoindoline-1,3-dione | 38.5% |
| 25 | 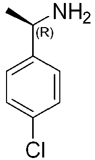 (R)-1-(4-chlorophenyl)ethan-1-amine | 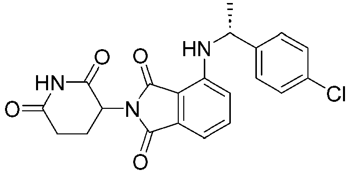 4-(((R)-1-(4-chlorophenyl)ethyl)amino)-2-(2,6-dioxopiperidin-3-yl)isoindoline-1,3-dione | 38.4% |
| 26 | 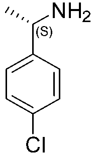 (S)-1-(4-chlorophenyl)ethan-1-amine | 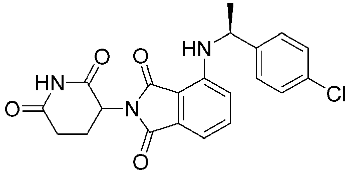 4-(((S)-1-(4-chlorophenyl)ethyl)amino)-2-(2,6-dioxopiperidin-3-yl)isoindoline-1,3-dione | 31% |
| 27 | 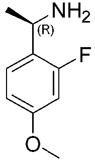 (R)-1-(2-fluoro-4-methoxyphenyl)ethan-1-amine | 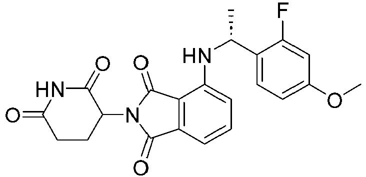 2-(2,6-dioxopiperidin-3-yl)-4-(((R)-1-(2-fluoro-4-methoxyphenyl)ethyl)amino)isoindoline -1,3-dione | 66% |
| 28 | 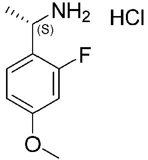 (S)-1-(2-fluoro-4-methoxyphenyl)ethan-1-amine Hydrochloride | 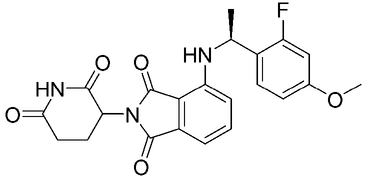 2-(2,6-dioxopiperidin-3-yl)-4-(((S)-1-(2-fluoro-4-methoxyphenyl)ethyl)amino)isoindoline -1,3-dione | 41% |
| 29 | 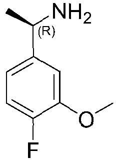 (R)-1-(4-fluoro-3-methoxyphenyl)ethan-1-amine | 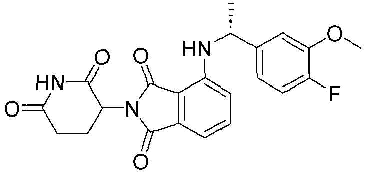 2-(2,6-dioxopiperidin-3-yl)-4-(((R)-1-(4-fluoro-3-methoxyphenyl)ethyl)amino)isoindoline -1,3-dione | 68% |
| 30 | 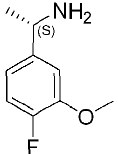 (S)-1-(4-fluoro-3-methoxyphenyl)ethan-1-amine | 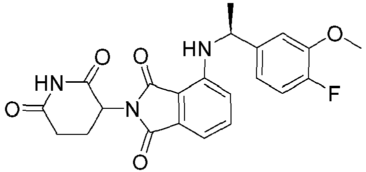 2-(2,6-dioxopiperidin-3-yl)-4-(((S)-1-(4-fluoro-3-methoxyphenyl)ethyl)amino)isoindoline -1,3-dione | 80% |
| 31 | 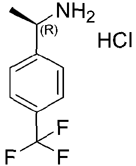 (R)-1-(4-(trifluoromethyl)phenyl) ethan-1-amine hydrochloride | 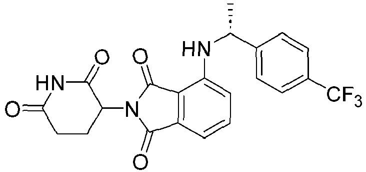 2-(2,6-dioxopiperidin-3-yl)-4-(((R)-1-(4-(trifluoromethyl)phenyl)ethyl)amino)is oindoline-1,3-dione | 70.8% |
| 32 | 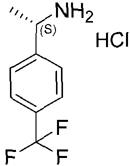 (S)-1-(4-(trifluoromethyl)phenyl) ethan-1-amine hydrochloride | 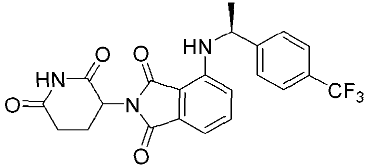 2-(2,6-dioxopiperidin-3-yl)-4-(((S)-1-(4-(trifluoromethyl)phenyl)ethyl)amino) isoindoline-1,3-dione | 48.4% |
| 33 | 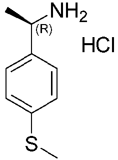 (R)-1-(4-(methylthio)phenyl) ethan-1-amine hydrochloride | 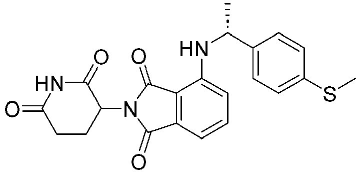 2-(2,6-dioxopiperidin-3-yl)-4-(((R)-1-(4-(methylthio)phenyl)ethyl)amino)isoind oline-1,3-dione | 74.4% |
| 34 | 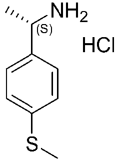 (S)-1-(4-(methylthio)phenyl) ethan-1-amine hydrochloride | 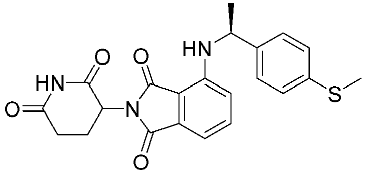 2-(2,6-dioxopiperidin-3-yl)-4-(((S)-1-(4-(methylthio)phenyl)ethyl)amino)isoind oline-1,3-dione | 50% |
| 35 | 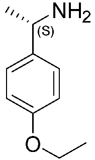 (S)-1-(4-ethoxyphenyl)ethan-1-amine | 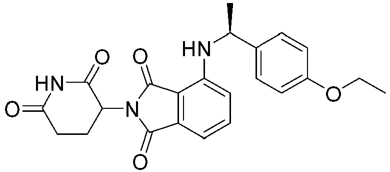 2-(2,6-dioxopiperidin-3-yl)-4-(((S)-1-(4-ethoxyphenyl)ethyl)amino)isoindoline-1,3-dione | 57.7% |
| 36 | 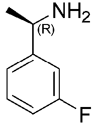 (R)-1-(3-fluorophenyl)ethan-1-amine | 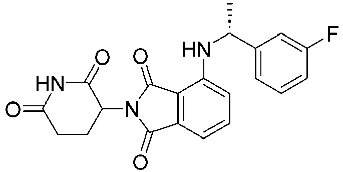 2-(2,6-dioxopiperidin-3-yl)-4-(((R)-1-(3-fluorophenyl)ethyl)amino)isoindoline-1,3-dione | 30% |
| 37 | 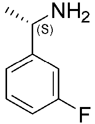 (S)-1-(3-fluorophenyl)ethan-1-amine | 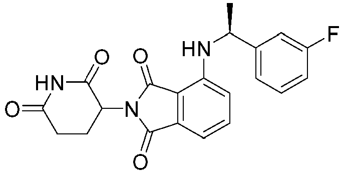 2-(2,6-dioxopiperidin-3-yl)-4-(((S)-1-(3-fluorophenyl)ethyl)amino)isoindoline-1,3-dione | 33% |
| 38 | 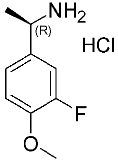 (R)-1-(3-fluoro-4-methoxyphenyl)ethan-1-amine hydrochloride | 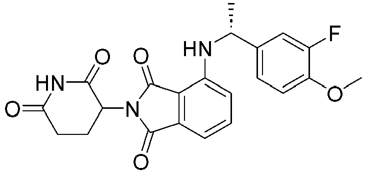 2-(2,6-dioxopiperidin-3-yl)-4-(((R)-1-(3-fluoro-4-methoxyphenyl)ethyl)amino)isoindoline -1,3-dione | 38% |
| 39 | 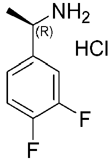 (R)-1-(3,4-difluorophenyl)ethan-1-amine hydrochloride | 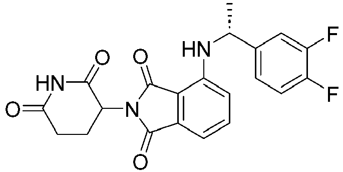 4-(((R)-1-(3,4-difluorophenyl)ethyl)amino)-2-(2,6-dioxopiperidin-3-yl)isoindoline-1,3-dione | 38% |
| 40 | 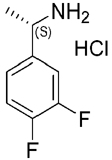 (S)-1-(3,4-difluorophenyl)ethan-1-amine hydrochloride | 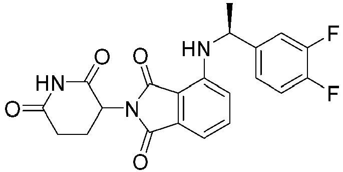 4-(((S)-1-(3,4-difluorophenyl)ethyl)amino)-2-(2,6-dioxopiperidin-3-yl)isoindoline-1,3-dione | 44% |
| 41 | 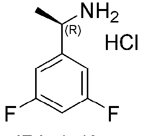 (R)-1-(3,5-difluorophenyl)ethan-1-amine hydrochloride | 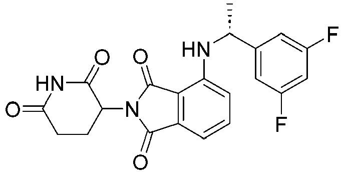 4-(((R)-1-(3,5-difluorophenyl)ethyl)amino)-2-(2,6-dioxopiperidin-3-yl)isoindoline-1,3-dione | 44% |
| 42 | 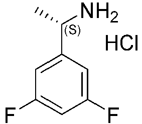 (S)-1-(3,5-difluorophenyl)ethan-1-amine hydrochloride | 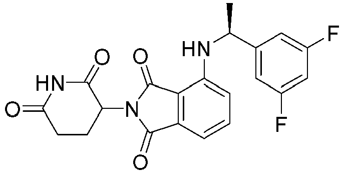 4-(((S)-1-(3,5-difluorophenyl)ethyl)amino)-2-(2,6-dioxopiperidin-3-yl)isoindoline-1,3-dione | 54% |
| 43 | 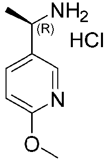 (R)-1-(6-methoxypyridin-3-yl)ethan-1-amine hydrochloride | 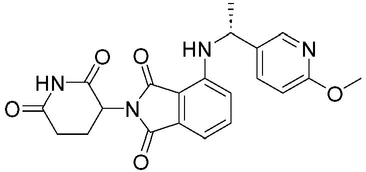 2-(2,6-dioxopiperidin-3-yl)-4-(((R)-1-(6-methoxypyridin-3-yl)ethyl)amino)isoindoline-1,3-dione | 49% |
| 44 | 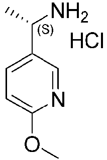 (S)-1-(6-methoxypyridin-3-yl)ethan-1-amine hydrochloride | 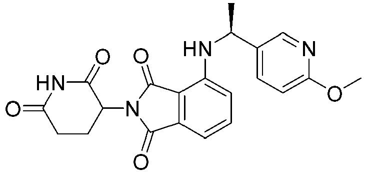 2-(2,6-dioxopiperidin-3-yl)-4-(((S)-1-(6-methoxypyridin-3-yl)ethyl)amino)isoindoline-1,3-dione | 24% |
| 45 | 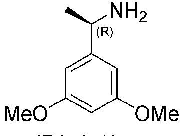 (R)-1-(3,5-dimethoxyphenyl)ethan -1-amine | 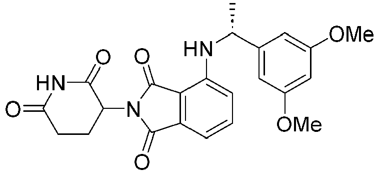 4-(((R)-1-(3,5-dimethoxyphenyl)ethyl)amino)-2-(2,6-dioxopiperidin-3-yl)isoindoline-1,3-dione | 64% |
| 46 | 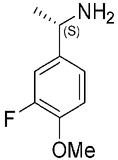 (S)-1-(3-fluoro-4-methoxyphenyl)ethan-1-amine | 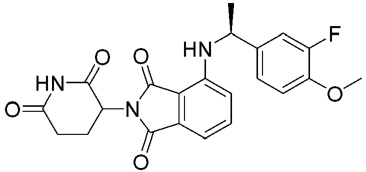 2-(2,6-dioxopiperidin-3-yl)-4-(((S)-1-(3-fluoro-4-methoxyphenyl)ethyl)amino)isoindoline -1,3-dione | 71% |
| 47 | 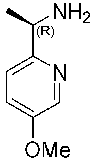 (R)-1-(5-methoxypyridin-2-yl)ethan-1-amine | 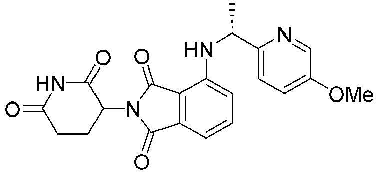 2-(2,6-dioxopiperidin-3-yl)-4-(((R)-1-(5-methoxypyridin-2-yl)ethyl)amino)isoindoline-1,3-dione | 38% |
| 48 | 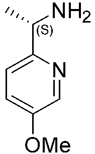 (S)-1-(5-methoxypyridin-2-yl)ethan-1-amine | 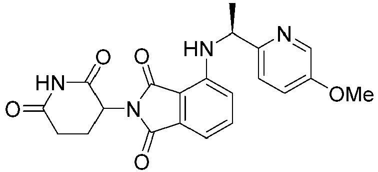 2-(2,6-dioxopiperidin-3-yl)-4-(((S)-1-(5-methoxypyridin-2-yl)ethyl)amino)isoindoline-1,3-dione | 54% |
| 49 | 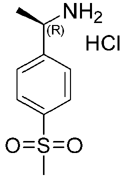 (R)-1-(4-(methylsulfonyl)phenyl) ethan-1-amine hydrochloride | 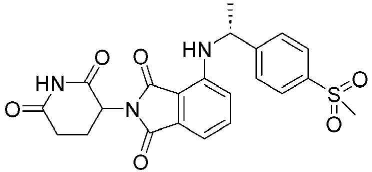 2-(2,6-dioxopiperidin-3-yl)-4-(((R)-1-(4-(methylsulfonyl)phenyl)ethyl)amino) isoindoline-1,3-dione | 58.2% |
| 50 | 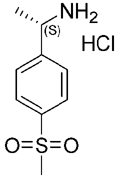 (S)-1-(4-(methylsulfonyl)phenyl) ethan-1-amine hydrochloride | 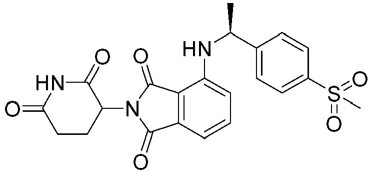 2-(2,6-dioxopiperidin-3-yl)-4-(((S)-1-(4-(methylsulfonyl)phenyl)ethyl)amino) isoindoline-1,3-dione | 52.7% |
| 51 | 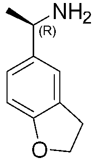 (R)-1-(2,3-dihydrobenzofuran-5-yl)ethan-1-amine | 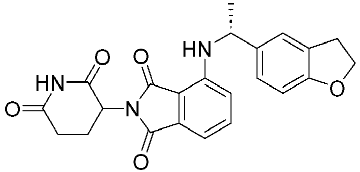 4-(((R)-1-(2,3-dihydrobenzofuran-5-yl)ethyl)amino)-2-(2,6-dioxopiperidin-3-yl)isoindoline-1,3-dione | 86% |
| 52 | 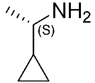 (S)-1-cyclopropylethan-1-amine | 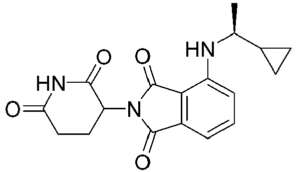 4-(((S)-1-cyclopropylethyl)amino)-2-(2,6-dioxopiperidin-3-yl)isoindoline-1,3-dione | 62% |

Table 2 below shows the NMR and MS analysis data on the compounds in Table 1 above.

**[Table 2]**

| **No.** | **Name** | **¹H NMR** (400 MHz) δ (ppm) | **MS** |
|---|---|---|---|
| 1 | methyl (3R)-3-((2-(2,6-dioxopiperidin-3-yl)-1,3-dioxoisoindolin-4-yl)amino) butanoate | (DMSO-d₆)11.10 (s, 1H), 7.64 (m, 1H), 7.46 (d, 1H), 7.11 (d, 1H), 6.41 (m, 1H), 5.04 (m, 1H), 4.93 (m, 1H), 3.58 (m, 1H), 3.49 (m, 2H), 2.87 (m, 1H), 2.60 (m, 1H), 2.04 (m, 1H), 1.67 (m, 1H), 1.50 (m, 1H), 0.92 (t, 3H) | 374(M+1) |
| 2 | methyl (3S)-3-((2-(2,6-dioxopiperidin-3-yl)-1,3-dioxoisoindolin-4-yl)amino) butanoate | (DMSO-d₆) 11.10 (s, 1H), 7.64 (m, 1H), 7.46 (d, 1H), 7.11 (d, 1H), 6.41 (m, 1H), 5.04 (m, 1H), 4.93 (m, 1H), 3.58 (m, 1H), 3.49 (m, 2H), 2.87 (m, 1H), 2.60 (m, 1H), 2.04 (m, 1H), 1.67 (m, 1H), 1.50 (m, 1H), 0.92 (t, 3H) | 374(M+1) |
| 3 | 2-(2,6-dioxopiperidin-3-yl)-4-(((S)-1-hydroxypropan-2-yl)amino)isoindoline -1,3-dione | (DMSO-d₆) 1.17(3H, d), 2.02(1H, m), 2.54(2H, m), 2.88(1H, m), 3.47(2H, m), 3.78(1H, m), 5.01(2H, m), 6.43(1H, m), 7.03(1H, d), 7.15(1H, d), 7.58(1H, m), 11.11(1H, s) | 332(M+1) |
| 4 | 2-(2,6-dioxopiperidin-3-yl)-4-(((R)-1-hydroxypropan-2-yl)amino)isoindoline -1,3-dione | (DMSO-d₆) 1.17(3H, d), 2.02(1H, m), 2.54(2H, m), 2.88(1H, m), 3.47(2H, m), 3.79(1H, m), 5.02(2H, m), 6.43(1H, m), 7.03(1H, d), 7.15(1H, d), 7.58(1H, m), 11.11(1H, s) | 332(M+1) |
| 5 | 2-(2,6-dioxopiperidin-3-yl)-4-(((S)-pentan-2-yl)amino)isoindoline-1,3-dione | (CDCl₃) 0.94(3H, t), 1.24(3H, d), 1.40(2H, m), 1.60(2H, m), 2.14(1H, m), 2.80(3H, m), 3.64(1H, m), 4.91(1H, m), 6.13(1H, br s), 6.88(1H, d), 7.06(1H, d), 7.47(1H, dd), 8.01(1H, br s) | 344(M+1) |
| 6 | 2-(2,6-dioxopiperidin-3-yl)-4-((3-methylbutan-2-yl)amino)isoindoline-1,3-dione | (CDCl₃) 1.00(6H, dd), 1.21(3H, d), 1.89(1H, m), 2.16(1H, m), 2.83(3H, m), 3.52(1H, m), 3.51(1H, m), 4.94(1H, m), 6.27(1H, m), 6.90(1H, d), 7.08(1H, d), 7.49(1H, dd), 8.10(1H, br s) | 344(M+1) |
| 7 | 4-((3,3-dimethylbutan-2-yl)amino)-2-(2,6-dioxopiperidin-3-yl)isoindoline-1,3-dione | (CDCl₃) 0.99(9H, s), 1.17(3H, d), 2.14(1H, m), 2.81 (3H, m), 3.42(1H, m), 4.92(1H, m), 6.35(1H, m), 6.91(1H, d), 7.04(1H, d), 7.46(1H, dd), 8.07(1H, br s) | 358(M+1) |
| 8 | 2-(2,6-dioxopiperidin-3-yl)-4-(((S)-1-methoxypropan-2-yl)amino)isoindoline-1,3-dione | (CDCl₃) 1.31(3H, d), 2.15(1H, m), 2.84(3H, m), 3.41(3H, s), 3.47(1H, m), 3.86(1H, m), 4.94(1H, m), 6.35(1H, d), 7.11(1H, d), 7.50(1H, m), 8.10(1H, br s) | 346(M+1) |
| 9 | 2-(2,6-dioxopiperidin-3-yl)-4-(((R)-1-(4-fluorophenyl)ethyl) amino)isoindoline-1,3-dione | (CDCl₃) 8.06 (s, 1H), 7.31 (m, 3H), 7.08 (m, 3H), 6.60 (d, 2H), 4.94 (m, 1H), 4.63 (m, 1H), 2.84 (m, 3H), 2.16 (m, 1H), 1.57 (d, 3H) | 396(M+1) |
| 10 | 2-(2,6-dioxopiperidin-3-yl)-4-(((S)-1-(4-fluorophenyl)ethyl) amino)isoindoline-1,3-dione | (CDCl₃) 8.06 (s, 1H), 7.31 (m, 3H), 7.08 (m, 3H), 6.60 (d, 2H), 4.94 (m, 1H), 4.63 (m, 1H), 2.84 (m, 3H), 2.16 (m, 1H), 1.57 (d, 3H). | 396(M+1) |
| 11 | 2-(2,6-dioxopiperidin-3-yl)-4-(((S)-1-(4-methoxyphenyl) ethyl)amino)isoindoline-1,3-dione | (CDCl₃) 1.57(3H, d), 2.15(1H, m), 2.84(3H, m), 3.79(3H, s), 4.60(1H, m), 4.94(1H, m), 6.59(1H, m), 6.66(1H, d), 6.88(2H, d), 7.07(1H, d), 7.26(2H, d), 7.34(1H, dd), 8.06(1H, br s) | 408(M+1) |
| 12 | 2-(2,6-dioxopiperidin-3-yl)-4-(((R)-1-(4-methoxyphenyl)ethyl) amino)isoindoline-1,3-dione | (CDCl₃) 1.60(3H, d), 2.17(1H, m), 2.85(3H, m), 3.81(3H, s), 4.62(1H, m), 4.96(1H, m), 6.61(1H, m), 6.68(1H, d), 6.89(2H, d), 7.09(1H, d), 7.28(2H, d), 7.36(1H, dd), 8.14(1H, br s) | 408(M+1) |
| 13 | 2-(2,6-dioxopiperidin-3-yl)-4-(((R)-1-(4-(trifluoromethoxy) phenyl)ethyl)amino)isoindo line-1,3-dione | (CDCl₃) 8.10 (s, 1H), 7.38 (m, 3H), 7.19 (d, 2H), 7.10 (d, 1H), 6.58 (d, 2H), 4.94 (m, 1H), 4.63 (m, 1H), 2.84 (m, 3H), 2.16 (m, 1H), 1.60 (d, 3H). | 462(M+1) |
| 14 | 2-(2,6-dioxopiperidin-3-yl)-4-(((S)-1-(4-trifluoromethoxy) phenyl)ethyl)amino)isoindo line-1,3-dione | (CDCl₃) 8.10 (s, 1H), 7.38 (m, 3H), 7.19 (d, 2H), 7.10 (d, 1H), 6.58 (d, 2H), 4.94 (m, 1H), 4.63 (m, 1H), 2.84 (m, 3H), 2.16 (m, 1H), 1.60 (d, 3H). | 462(M+1) |
| 15 | 2-(2,6-dioxopiperidin-3-yl)-4-(((R)-1-(2-fluorophenyl)ethyl) amino)isoindoline-1,3-dione | (CDCl₃) 8.13 (s, 1H), 7.35 (m, 2H), 7.09 (m, 2H), 7.10 (d, 3H), 6.65 (d, 1H), 6.60(m, 1H), 4.94 (m, 2H), 2.84 (m, 3H), 2.16 (m, 1H), 1.60 (d, 3H). | 396(M+1) |
| 16 | 2-(2,6-dioxopiperidin-3-yl)-4-(((S)-1-(2-fluorophenyl)ethyl) amino)isoindoline-1,3-dione | (CDCl₃) 8.13 (s, 1H), 7.35 (m, 2H), 7.09 (m, 2H), 7.10 (d, 3H), 6.65 (d, 1H), 6.60(m, 1H), 4.94 (m, 2H), 2.84 (m, 3H), 2.16 (m, 1H), 1.60 (d, 3H). | 396(M+1) |
| 17 | 2-(2,6-dioxopiperidin-3-yl)-4-(((R)-1-(3-methoxyphenyl) ethyl)amino)isoindoline-1,3-dione | (CDCl₃) 8.05 (s, 1H), 7.33 (m, 1H), 7.08 (d, 1H), 6.93 (d, 1H), 6.88 (m, 1H), 6.80 (dd, 1H), 6.65 (dd, 1H), 6.60 (m, 1H), 4.95 (m, 1H), 4.59 (m, 1H), 3.80 (s, 3H), 2.84 (m, 3H), 2.16 (m, 1H), 1.57 (d, 3H). | 408(M+1) |
| 18 | 2-(2,6-dioxopiperidin-3-yl)-4-(((S)-1-(3-methoxyphenyl) ethyl)amino)isoindoline-1,3-dione | (CDCl₃) 8.05 (s, 1H), 7.33 (m, 1H), 7.08 (d, 1H), 6.93 (d, 1H), 6.88 (m, 1H), 6.80 (dd, 1H), 6.65 (dd, 1H), 6.60 (m, 1H), 4.95 (m, 1H), 4.59 (m, 1H), 3.80 (s, 3H), 2.84 (m, 3H), 2.16 (m, 1H), 1.57 (d, 3H). | 408(M+1) |
| 19 | 2-(2,6-dioxopiperidin-3-yl)-4-(((R)-1-(2-(trifluoromethyl) phenyl)ethyl)amino)isoindo line-1,3-dione | (CDCl₃) 8.06 (s, 1H), 7.70 (d, 1H), 7.52 (t, 1H), 7.35 (m, 2H), 7.07 (d, 1H), 6.72 (m, 1H), 6.60(d, 1H), 5.05 (m, 1H), 4.96 (m, 1H), 2.84 (m, 3H), 2.17 (m, 1H), 1.60 (d, 3H). | 446(M+1) |
| 20 | 4-((1R)-1-((2-(2,6-dioxopiperidin-3-yl)-1,3-dioxo isoindolin-4-yl)amino)ethyl) benzenesulfonamide | (CDCl₃) 8.12 (s, 1H), 7.91 (d, 2H), 7.52 (d, 2H), 7.35 (t, 1H), 7.12 (d, 1H), 6.64 (m, 1H), 6.52 (d, 1H), 4.96 (m, 1H), 4.85 (s, 2H), 4.71 (m, 1H), 2.84 (m, 3H), 2.18 (m, 1H), 1.60 (d, 3H). | 457(M+1) |
| 21 | methyl 4-((1R)-1-((2-(2,6-diox opiperidin-3-yl)-1,3-dioxoiso indolin-4-yl)amino)ethyl)ben zoate | (CDCl₃) 8.11 (s, 1H), 8.02 (d, 2H), 7.42 (d, 2H), 7.32 (t, 1H), 7.09 (d, 1H), 6.63 (m, 1H), 6.54 (d, 1H), 4.96 (m, 1H), 4.68 (m, 1H), 2.84 (m, 3H), 2.16 (m, 1H), 1.61 (d, 3H). | 436(M+1) |
| 22 | methyl 4-((1S)-1-((2-(2,6-dioxo piperidin-3-yl)-1,3-dioxoisoind olin-4-yl)amino)ethyl)benzoate | (CDCl₃) 8.11 (s, 1H), 8.02 (d, 2H), 7.42 (d, 2H), 7.32 (t, 1H), 7.09 (d, 1H), 6.63 (m, 1H), 6.54 (d, 1H), 4.96 (m, 1H), 4.68 (m, 1H), 2.84 (m, 3H), 2.16 (m, 1H), 1.61 (d, 3H). | 436(M+1) |
| 23 | 2-(2,6-dioxopiperidin-3-yl)-4-(((R)-1-(p-tolyl)ethyl)amino)iso indoline-1,3-dione | (CDCl₃) 8.05 (s, 1H), 7.33 (t, 1H), 7.23 (d, 2H), 7.15 (d, 2H), 7.00 (d, 1H), 6.65 (d, 1H), 6.50 (m, 1H), 4.96 (m, 1H), 4.60 (m, 1H), 2.84 (m, 3H), 2.33 (s, 3H), 2.16 (m, 1H), 1.61 (d, 3H). | 392(M+1) |
| 24 | 2-(2,6-dioxopiperidin-3-yl)-4-(((S)-1-(p-tolyl)ethyl)amino)iso indoline-1,3-dione | (CDCl₃) 8.05 (s, 1H), 7.33 (t, 1H), 7.23 (d, 2H), 7.15 (d, 2H), 7.00 (d, 1H), 6.65 (d, 1H), 6.50 (m, 1H), 4.96 (m, 1H), 4.60 (m, 1H), 2.84 (m, 3H), 2.33 (s, 3H), 2.16 (m, 1H), 1.61 (d, 3H). | 392(M+1) |
| 25 | 4-(((R)-1-(4-chlorophenyl)ethyl) amino)-2-(2,6-dioxopiperidin-3-yl)isoindoline-1,3-dione | (CDCl₃) 8.07 (s, 1H), 7.33 (m, 4H), 7.10 (d, 1H), 6.58 (m, 2H), 4.96 (m, 1H), 4.62 (m, 1H), 2.84 (m, 3H), 2.33 (s, 3H), 2.16 (m, 1H), 1.57 (d, 3H). | 412(M+1) |
| 26 | 4-(((S)-1-(4-chlorophenyl)ethyl) amino)-2-(2,6-dioxopiperidin-3-yl)isoindoline-1,3-dione | (CDCl₃) 8.07 (s, 1H), 7.33 (m, 4H), 7.10 (d, 1H), 6.58 (m, 2H), 4.96 (m, 1H), 4.62 (m, 1H), 2.84 (m, 3H), 2.33 (s, 3H), 2.16 (m, 1H), 1.57 (d, 3H). | 412(M+1) |
| 27 | 2-(2,6-dioxopiperidin-3-yl)-4-(((R)-1-(2-fluoro-4-ethoxyphe nyl)ethyl)amino)isoindoline -1,3-dione | (CDCl₃) 8.05 (s, 1H), 7.37 (t, 1H), 7.21 (t, 1H), 7.08 (d, 1H), 6.65 (m, 3H), 6.56 (m, 1H), 4.91 (m, 2H), 3.78 (s, 3H), 2.84 (m, 3H), 2.16 (m, 1H), 1.59 (d, 3H). | 426(M+1) |
| 28 | 2-(2,6-dioxopiperidin-3-yl)-4-(((S)-1-(2-fluoro-4-methoxy phenyl)ethyl)amino)isoindo line-1,3-dione | (CDCl₃) 8.05 (s, 1H), 7.37 (t, 1H), 7.21 (t, 1H), 7.08 (d, 1H), 6.65 (m, 3H), 6.56 (m, 1H), 4.91 (m, 2H), 3.78 (s, 3H), 2.84 (m, 3H), 2.16 (m, 1H), 1.59 (d, 3H). | 426(M+1) |
| 29 | 2-(2,6-dioxopiperidin-3-yl)-4-(((R)-1-(4-fluoro-3-methoxy phenyl)ethyl)amino)isoindoline-1,3-dione | (CDCl₃) 8.19 (s, 1H), 7.35 (t, 1H), 7.10 (d, 1H), 7.04 (m, 1H), 6.92 (dd, 1H), 6.88 (m, 1H), 6.62 (d, 1H), 6.58 (m, 1H), 4.96 (m, 1H), 4.60 (m, 1H), 3.88 (s, 3H), 2.84 (m, 3H), 2.33 (s, 3H), 2.16 (m, 1H), 1.59 (d, 3H). | 426(M+1) |
| 30 | 2-(2,6-dioxopiperidin-3-yl)-4-(((S)-1-(4-fluoro-3-methoxy phenyl)ethyl)amino)isoindo line-1,3-dione | (CDCl₃) 8.19 (s, 1H), 7.35 (t, 1H), 7.10 (d, 1H), 7.04 (m, 1H), 6.92 (dd, 1H), 6.88 (m, 1H), 6.62 (d, 1H), 6.58 (m, 1H), 4.96 (m, 1H), 4.60 (m, 1H), 3.88 (s, 3H), 2.84 (m, 3H), 2.33 (s, 3H), 2.16 (m, 1H), 1.59 (d, 3H). | 426(M+1) |
| 31 | 2-(2,6-dioxopiperidin-3-yl)-4-(((R)-1-(4-(trifluoromethyl) phenyl)ethyl)amino)isoindo line-1,3-dione | (CDCl₃) 8.04 (s, 1H), 7.61 (d, 2H), 7.47 (d, 2H), 7.35 (t, 1H), 7.11 (d, 1H), 6.63 (m, 1H), 6.64 (d, 1H), 4.96 (m, 1H), 4.69 (m, 1H), 2.84 (m, 3H), 2.16 (m, 1H), 1.62 (d, 3H). | 446(M+1) |
| 32 | 2-(2,6-dioxopiperidin-3-yl)-4-(((S)-1-(4-(trifluoromethyl) phenyl)ethyl)amino)isoindo line-1,3-dione | (CDCl₃) 8.04 (s, 1H), 7.61 (d, 2H), 7.47 (d, 2H), 7.35 (t, 1H), 7.11 (d, 1H), 6.63 (m, 1H), 6.64 (d, 1H), 4.96 (m, 1H), 4.69 (m, 1H), 2.84 (m, 3H), 2.16 (m, 1H), 1.62 (d, 3H). | 446(M+1) |
| 33 | 2-(2,6-dioxopiperidin-3-yl)-4-(((R)-1-(4-(methylthio)phenyl) ethyl)amino)isoindoline-1,3-dione | (CDCl₃) 8.10 (s, 1H), 7.35 (t, 1H), 7.25 (m, 4H), 7.07 (d, 1H), 6.60 (m, 2H), 4.94 (m, 1H), 4.59 (m, 1H), 2.84 (m, 3H), 2.48 (s, 3H), 2.16 (m, 1H), 1.61 (d, 3H). | 424(M+1) |
| 34 | 2-(2,6-dioxopiperidin-3-yl)-4-(((S)-1-(4-(methylthio)phenyl) ethyl)amino)isoindoline-1,3-dione | (CDCl₃) 8.10 (s, 1H), 7.35 (t, 1H), 7.25 (m, 4H), 7.07 (d, 1H), 6.60 (m, 2H), 4.94 (m, 1H), 4.59 (m, 1H), 2.84 (m, 3H), 2.48 (s, 3H), 2.16 (m, 1H), 1.61 (d, 3H). | 424(M+1) |
| 35 | 2-(2,6-dioxopiperidin-3-yl)-4-(((S)-1-(4-ethoxyphenyl)ethyl) amino)isoindoline-1,3-dione | (CDCl₃) 8.04 (s, 1H), 7.33 (t, 1H), 7.25 (d, 2H), 7.07 (d, 1H), 6.88 (d, 2H), 6.68 (d, 1H), 6.65 (m, 1H), 4.94 (m, 1H), 4.59 (m, 1H), 4.00 (q, 2H), 2.84 (m, 3H), 2.48 (s, 3H), 2.16 (m, 1H), 1.61 (d, 3H), 1.40 (t, 3H). | 422(M+1) |
| 36 | 2-(2,6-dioxopiperidin-3-yl)-4-(((R)-1-(3-fluorophenyl)ethyl)amino)isoindoline-1,3-dione | (CDCl₃) 8.01 (s, 1H), 7.33 (m, 2H), 7.12 (m, 2H), 7.08 (m, 1H), 6.95 (m, 1H), 6.61 (m, 2H), 4.95 (m, 1H), 4.62 (m, 1H), 2.84 (m, 3H), 2.16 (m, 1H), 1.61 (d, 3H). | 396(M+1) |
| 37 | 2-(2,6-dioxopiperidin-3-yl)-4-(((S)-1-(3-fluorophenyl)ethyl) amino)isoindoline-1,3-dione | (CDCl₃) 8.01 (s, 1H), 7.33 (m, 2H), 7.12 (m, 2H), 7.08 (m, 1H), 6.95 (m, 1H), 6.61 (m, 2H), 4.95 (m, 1H), 4.62 (m, 1H), 2.84 (m, 3H), 2.16 (m, 1H), 1.61 (d, 3H). | 396(M+1) |
| 38 | 2-(2,6-dioxopiperidin-3-yl)-4-(((R)-1-(3-fluoro-4-methoxyphe nyl)ethyl)amino)isoindoline -1,3-dione | (CDCl₃) 8.02 (s, 1H), 7.35 (t, 1H), 7.08 (m, 3H), 6.91 (m, 1H), 6.62 (d, 1H), 6.56 (m, 1H), 4.95 (m, 1H), 4.57 (m, 1H), 3.87 (s, 3H), 2.84 (m, 3H), 2.16 (m, 1H), 1.61 (d, 3H). | 414(M+1) |
| 39 | 4-(((R)-1-(3,4-difluorophenyl) ethyl)amino)-2-(2,6-dioxopipe ridin-3-yl)isoindoline-1,3-dione | (CDCl₃) 8.12 (s, 1H), 7.36 (t, 1H), 7.15 (m, 4H), 6.65 (m, 2H), 4.96 (m, 1H), 4.59 (m, 1H), 2.84 (m, 3H), 2.16 (m, 1H), 1.61 (d, 3H). | 414(M+1) |
| 40 | 4-(((S)-1-(3,4-difluorophenyl) ethyl)amino)-2-(2,6-dioxopipe ridin-3-yl)isoindoline-1,3-dione | (CDCl₃) 8.12 (s, 1H), 7.36 (t, 1H), 7.15 (m, 4H), 6.65 (m, 2H), 4.96 (m, 1H), 4.59 (m, 1H), 2.84 (m, 3H), 2.16 (m, 1H), 1.61 (d, 3H). | 414(M+1) |
| 41 | 4-(((R)-1-(3,5-difluorophenyl) ethyl)amino)-2-(2,6-dioxopipe ridin-3-yl)isoindoline-1,3-dione | (CDCl₃) 8.15 (s, 1H), 7.37 (t, 1H), 7.10 (d, 1H), 6.86 (m, 2H), 6.70 (m, 1H), 6.57 (m, 2H), 4.96 (m, 1H), 4.59 (m, 1H), 2.84 (m, 3H), 2.16 (m, 1H), 1.61 (d, 3H). | 414(M+1) |
| 42 | 4-(((S)-1-(3,5-difluorophenyl) ethyl)amino)-2-(2,6-dioxopipe ridin-3-yl)isoindoline-1,3-dione | (CDCl₃) 8.15 (s, 1H), 7.37 (t, 1H), 7.10 (d, 1H), 6.86 (m, 2H), 6.70 (m, 1H), 6.57 (m, 2H), 4.96 (m, 1H), 4.59 (m, 1H), 2.84 (m, 3H), 2.16 (m, 1H), 1.61 (d, 3H). | 414(M+1) |
| 43 | 2-(2,6-dioxopiperidin-3-yl)-4-(((R)-1-(6-methoxypyridin-3-yl)ethyl)amino)isoindoline-1,3-dione | (CDCl₃) 8.16 (d, 1H), 8.12 (s, 1H), 7.57 (dd, 1H), 7.37 (t, 1H), 7.10 (d, 1H), 6.74 (d, 1H), 6.67 (d, 1H), 6.56 (m, 1H), 4.96 (m, 1H), 4.64 (m, 1H), 2.84 (m, 3H), 2.16 (m, 1H), 1.61 (d, 3H). | 409(M+1) |
| 44 | 2-(2,6-dioxopiperidin-3-yl)-4-(((S)-1-(6-methoxypyridin-3-yl)ethyl)amino)isoindoline-1,3-dione | (CDCl₃) 8.16 (d, 1H), 8.12 (s, 1H), 7.57 (dd, 1H), 7.37 (t, 1H), 7.10 (d, 1H), 6.74 (d, 1H), 6.67 (d, 1H), 6.56 (m, 1H), 4.96 (m, 1H), 4.64 (m, 1H), 2.84 (m, 3H), 2.16 (m, 1H), 1.61 (d, 3H). | 409(M+1) |
| 45 | 4-(((R)-1-(3,5-dimethoxyphenyl) ethyl)amino)-2-(2,6-dioxopipe ridin-3-yl)isoindoline-1,3-dione | (CDCl₃) 8.08 (s, 1H), 7.35 (t, 1H), 7.08 (m, 3H), 6.66 (dd, 1H), 6.56 (m, 1H), 6.49 (m, 2H), 6.35(m, 1H), 4.95 (m, 1H), 4.53 (m, 1H), 3.79 (s, 6H), 2.84 (m, 3H), 2.16 (m, 1H), 1.57 (d, 3H). | 438(M+1) |
| 46 | 2-(2,6-dioxopiperidin-3-yl)-4-(((S)-1-(3-fluoro-4-methoxy phenyl)ethyl)amino)isoindo line-1,3-dione | (CDCl₃) 8.13 (s, 1H), 7.35 (t, 1H), 7.08 (m, 3H), 6.92 (m, 1H), 6.63 (d, 1H), 6.56 (m, 1H), 4.95 (m, 1H), 4.57 (m, 1H), 3.87 (s, 3H), 2.84 (m, 3H), 2.16 (m, 1H), 1.58 (d, 3H). | 426(M+1) |
| 47 | 2-(2,6-dioxopiperidin-3-yl)-4-(((R)-1-(5-methoxypyridin-2-yl)ethyl)amino)isoindoline-1,3-dione | (CDCl₃) 8.30 (d, 1H), 8.14 (s, 1H), 7.37 (t, 1H), 7.21 (d, 1H), 7.17 (dd, 1H), 7.09 (d, 1H), 6.86 (m, 1H), 6.73 (d, 1H), 4.96 (m, 1H), 4.77 (m, 1H), 3.85 (s, 3H), 2.84 (m, 3H), 2.16 (m, 1H), 1.61 (d, 3H). | 409(M+1) |
| 48 | 2-(2,6-dioxopiperidin-3-yl)-4-(((S)-1-(5-methoxypyridin-2-yl)ethyl)amino)isoindoline-1,3-dione | (CDCl₃) 8.30 (d, 1H), 8.14 (s, 1H), 7.37 (t, 1H), 7.21 (d, 1H), 7.17 (dd, 1H), 7.09 (d, 1H), 6.86 (m, 1H), 6.73 (d, 1H), 4.96 (m, 1H), 4.77 (m, 1H), 3.85 (s, 3H), 2.84 (m, 3H), 2.16 (m, 1H), 1.61 (d, 3H). | 409(M+1) |
| 49 | 2-(2,6-dioxopiperidin-3-yl)-4-(((R)-1-(4-(methylsulfonyl) phenyl)ethyl)amino)isoindo line-1,3-dione | (CDCl₃) 8.22 (s, 1H), 7.92 (d, 2H), 7.56 (d, 2H), 7.34 (t, 1H), 7.11 (d, 1H), 6.64 (m, 1H), 6.51 (dd, 1H), 4.96 (m, 1H), 4.72 (m, 1H), 3.06 (s, 3H), 2.84 (m, 3H), 2.16 (m, 1H), 1.61 (d, 3H). | 456(M+1) |
| 50 | 2-(2,6-dioxopiperidin-3-yl)-4-(((S)-1-(4-(methylsulfonyl) phenyl)ethyl)amino)isoindoline-1,3-dione | (CDCl₃) 8.22 (s, 1H), 7.92 (d, 2H), 7.56 (d, 2H), 7.34 (t, 1H), 7.11 (d, 1H), 6.64 (m, 1H), 6.51 (dd, 1H), 4.96 (m, 1H), 4.72 (m, 1H), 3.06 (s, 3H), 2.84 (m, 3H), 2.16 (m, 1H), 1.61 (d, 3H). | 456(M+1) |
| 51 | 4-(((R)-1-(2,3-dihydrobenzo furan-5-yl)ethyl)amino)-2-(2,6-dioxopiperidin-3-yl)isoindoline-1,3-dione | (CDCl₃) 8.11 (s, 1H), 7.34 (t, 1H), 7.17 (s, 1H), 7.07 (m, 2H), 6.74 (d, 1H), 6.68 (d, 1H), 6.51 (m, 1H), 4.94 (m, 1H), 4.58 (m, 3H), 3.18 (t, 2H), 2.84 (m, 3H), 2.16 (m, 1H), 1.61 (d, 3H). | 420(M+1) |
| 52 | 4-(((S)-1-cyclopropylethyl)ami no)-2-(2,6-dioxopiperidin-3-yl)isoindoline-1,3-dione | (CDCl₃) 8.04 (s, 1H), 7.46 (t, 1H), 7.07 (d, 1H), 6.86 (d, 1H), 6.26 (s, 1H), 4.92 (m, 1H), 3.18 (m, 1H), 2.84 (m, 3H), 2.16 (m, 1H), 1.30 (d, 3H), 1.00 (m, 1H), 0.55 (m, 2H), 0.30 (m, 2H). | 342(M+1) |

### Experimental Examples

### Experimental Example 1. Microbial Mutagenicity Assay - Ames Test

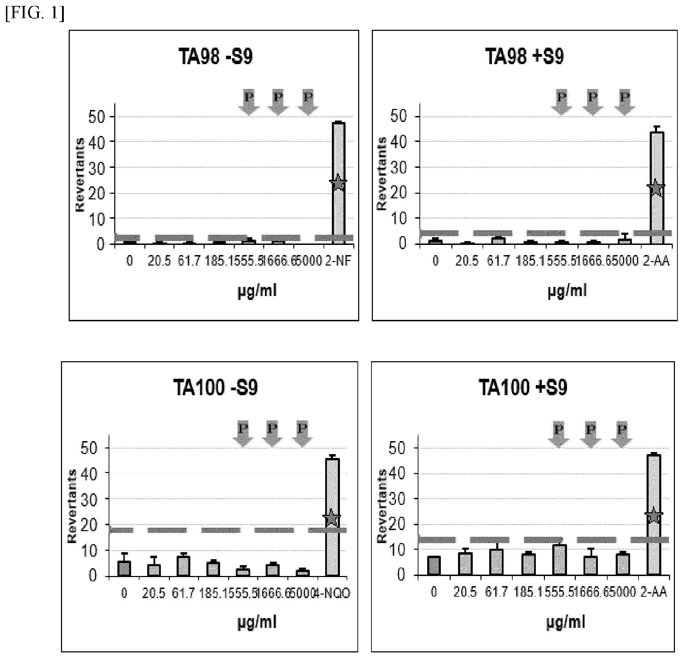

In order to evaluate the safety of the compound (AST-DT-218) prepared in Example above, the possibility of inducing genetic mutations was confirmed. First, TA98 and TA100 strains, which detect frameshift mutations and point mutations, respectively, were cultured. Then, AST-DT-218 was dissolved in a solvent and diluted to various concentrations (20.5 µg/mL, 61.7 µg/mL 185.1 µg/mL, 1555.5 µg/mL, 1666.6 µg/mL, and 5000 µg/mL) to prepare an S9 mixture (a metabolic activation system; concentration: 30%) including liver enzyme extracts and supplements. Into a histidine-deficient medium at different concentrations of AST-DT-218, (i) the TA98 strain was inoculated without the S9 mixture (TA98-S9), (ii) the TA98 strain was inoculated by mixing it with the S9 mixture (TA98+S9), (iii) the TA100 strain was inoculated without the S9 mixture (TA100-S9), and (iv) the TA100 strain was inoculated by mixing it with the S9 mixture (TA100+S9). As a solvent control, which is a negative control, only the solvent was inoculated into a histidine-deficient medium, and only a pure solvent was used in the absence of any compound. As a positive control, 2-nitrofluorene (2-NF), 2-aminoanthracene (2-AA), or 4-nitroquinoline 1-oxide (4-NQO) was inoculated into a histidine-deficient medium. After culturing for 48 to 72 hours in each medium, the number of colonies generated was counted to calculate the mutation rate, and the mutagenic effect was compared depending on the presence or absence of the S9 mixture to analyze the effect on metabolic activation. The results are shown in Table 3 and FIG. 1.

**[Table 3]**

| Strain | Test Conc. | **Mutagenic Data Points** | | **Overall Result** | Solvent Control | | Positive Control | |
|---|---|---|---|---|---|---|---|---|
| | | **w/o S9** | **w/ S9** | | w/o S9 | w/ S9 | w/o S9 | w/ S9 |
| TA98 | 20.5-5,000 µg/mL | **No** | **No** | **probably not mutagenic** | PASS | PASS | PASS | PASS |
| TA100 | | **No** | **No** | **probably not mutagenic** | PASS | PASS | PASS | PASS |

While a significant increase in the number of mutant colonies was observed in all positive controls (2-NF, 2-AA, and 4-NQO), no significant increase in the number of mutant colonies was observed for both TA98 and TA100 strains in the AST-DT-218 treatment group within the concentration range of experimental conditions, regardless of the presence or absence of the S9 mixture. Therefore, it was confirmed that AST-DT-218 does not induce genetic mutations.

### Experimental Example 2. hERG-Related Cardiotoxicity Test and Ligand Binding Assay

In order to evaluate the inhibitory effect of AST-DT-218 on the hERG channel and determine its potential to induce cardiotoxicity, a hERG-related cardiotoxicity test using an automated patch clamp was performed. First, a hERG channel-expressing cell line (CHO) was cultured, and AST-DT-218 was dissolved in a solvent (DMSO) and diluted to a concentration (1 µM). Cells were mounted on a patch-clamp device, and the electrical activity of hERG channels was recorded by measuring voltage-dependent currents. The inhibitory effect was calculated as the current reduction ratio, and the IC₅₀ value was derived by constructing a concentration-response curve.

Additionally, a ligand binding assay was performed to measure the affinity of AST-DT-218 that binds to the hERG channel. First, a hERG channel-expressing cell line (HEK293) was cultured, and then the cells were centrifuged to extract the cell membrane, and membrane proteins were prepared using a cell lysis reagent. A labeled hERG ligand was prepared and AST-DT-218 was dissolved in a solvent (DMSO) and diluted to a concentration (1 µM). After dispensing the cell membrane proteins into a 96-well plate or appropriate experimental equipment, competitive binding experiments were performed by adding AST-DT-218 at various concentrations to each well and incubated for 1 to 2 hours. After completion of the binding, the unbound ligands were washed using a recovery solution to separate the same, the extent of ligand binding was measured using a radiation detection device or fluorescence reader, and the signal intensities were analyzed to calculate the ligand binding inhibition ratio. The degree of binding inhibition according to the concentration of AST-DT-218 was plotted to derive the IC₅₀ value.

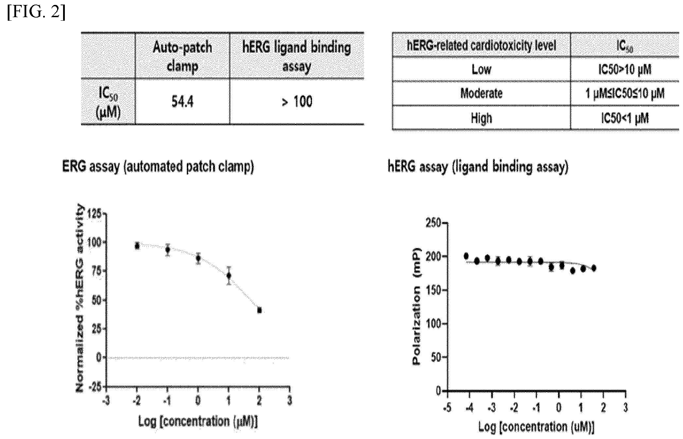

The results of the hERG-related cardiotoxicity test and ligand binding assay are shown in FIG. 2. From the results, it was confirmed that AST-DT-218 has a low possibility of causing cardiotoxicity.

### Experimental Example 3. Single Dose Toxicity Study

The toxicity of AST-DT-218 at a single dose was evaluated. First, healthy adult male and female rats were prepared and then fasted for 12 hours before the experiment. Solutions were prepared for each dose group (0 mg/kg, 500 mg/kg, 1,000 mg/kg, and 2,000 mg/kg), and the final solution volume was set at 40 mL/kg. Rats were randomly divided into groups of 5 males and 5 females into groups of G1 to G4 as shown in Table 4, and then each rat was given a single oral administration (p.o.) of 40 mL/kg.

**[Table 4]**

| Group | Gender | N | Rat Number (Male/Female) | Route | Dose (mg/kg) | Volume (mL/kg) |
|---|---|---|---|---|---|---|
| G1 | male/female | 5/5 | 1-5 / 21-25 | p.o. | 0 | 40 |
| G2 | male/female | 5/5 | 6-10 / 26-30 | p.o. | 500 | 40 |
| G3 | male/female | 5/5 | 11-15 / 31-35 | p.o. | 1,000 | 40 |
| G4 | male/female | 5/5 | 16-20 / 36-40 | p.o. | 2,000 | 40 |

For 1 to 15 days after administration, clinical symptoms (*e.g.*, colored stool, hair loss, *etc*.), changes in weight, changes in food consumption, and changes in organ weight were observed, and ophthalmic examinations were performed and autopsies were performed. The results are shown in Table 5.

**[Table 5]**

| Group | Animal ID | Clinical Signs | Day |
|---|---|---|---|
| G1 (0 mg/kg) | - | - | - |
| G2 (500 mg/kg) | all | colored stool | 2 |
| G3 (1,000 mg/kg) | all | colored stool | 2 |
| G4 (2,000 mg/kg) | all | colored stool | 2 |
| G4 (2,000 mg/kg) | #18 to #20 | colored stool | 3 |
| | #19 | loss of fur | 14-15 |
| | #40 | colored stool | 3 |

| | | | |
|---|---|---|---|
| -: None | | | |

There were no toxicologically significant changes in clinical symptoms, body weight, food consumption, ophthalmological examination, organ weights, and autopsies, when AST-DT-218 was administered as a single dose.

### Experimental Example 4. Maximum Tolerated Dose (MTD) Test

The *in vivo* maximum tolerated dose (MTD) of AST-DT-218 was evaluated to determine the highest dose at which AST-DT-218 could be safely administered to experimental animals. First, the compound AST-DT-218 and the preparation solvent to be used in the experiment were prepared. The solvent was a mixture of 5% Tween 80, 40% PEG400, 10% DMSO, and 45% DPBS, and AST-DT-218 was prepared according to each dose as shown in Table 6 below. Mice (n = 5) to be used in the experiment were prepared and divided into five groups to perform the experiment. The control group was injected with only 0 mg/kg of the solvent, and the remaining groups were injected with the AST-DT-218 solution at 2.5 mg/kg, 5 mg/kg, 10 mg/kg, and 20 mg/kg, respectively, and the injection amount was set to 0.1 mL for a 20 g mouse.

**[Table 6]**

| Condition (AST-DT-218) | Injection Volume (per 20 g of mouse) | Molecular Weight | AST-DT-218 (mM) |
|---|---|---|---|
| Control (0 mg/kg) | | | 0 mM |
| 2.5 mg/kg | | | ~ 1.25 mM (= 1.24 mM) |
| 5 mg/kg | 0.1 mL | 407.4192 g/mol | ~ 2.5 mM (= 2.48 mM) |
| 10 mg/kg | | | ~ 5 mM (= 4.98 mM) |
| 20 mg/kg | | | ~ 10 mM (= 9.95 mM) |

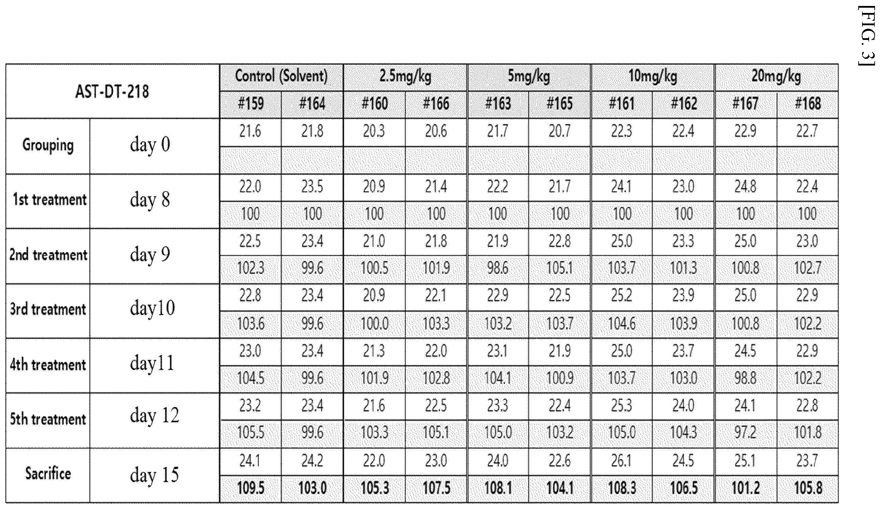

From day 1 to day 5, the injections were administered at the same time daily, and the changes in weight and abnormal behaviors of the mice were observed and data were collected during the experiment. The changes in weight of the mice are shown in FIG. 3. As shown in FIG. 3, it was shown to be non-toxic under solvent conditions, and no weight loss or abnormal behavior was observed even at the highest treatment concentration of 20 mg/kg.

### Experimental Example 5. Evaluation of treatment potential of hepatocellular carcinoma

An indication expansion experiment was performed to evaluate the potential of AST-DT-218 for treating hepatocellular carcinoma (HCC). First, HCC cell lines (Huh7, Hep3B, SNU449, and SNU475) were prepared, and AST-DT-218 and Sorafenib were at the highest concentration of 50 µM, serially diluted by half starting therefrom, thereby preparing 10 concentrations (50 µM, 25 µM, 12.5 µM, *etc*.). HCC cell lines (Huh7, Hep3B, SNU449, SNU475) were seeded into 96-well plates, in which each well was treated with AST-DT-218 and Sorafenib solutions, and the cells were cultured in an incubator for 24 hours. Then, the cell survival at each concentration was evaluated by measuring the cell count using cell nuclear staining, and a concentration-response curve of cell survival was created based on the data, and the IC₅₀ value was derived.

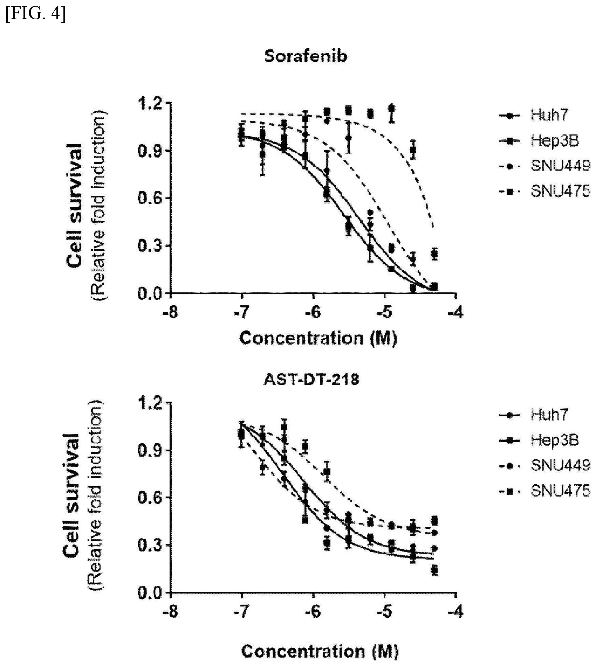

The experimental results are shown in Table 7 and FIG. 4. It was confirmed that AST-DT-218 showed significantly improved efficacy than Sorafenib in liver cancer.

**[Table 7]**

| | Hepatocellular Carcinoma Cell Line | | | |
|---|---|---|---|---|
| Compound (IC₅₀ (µM)) | Huh7 | Hep3B | SNU449 | SNU475 |
| AST-DT-218 | **0.75** | **0.378** | **0.155** | **1.449** |
| Sorafenib (HCC) | 4.368 | 5.675 | 11.280 | - |

### Experimental Example 6. Dose Response Curve (DRC)

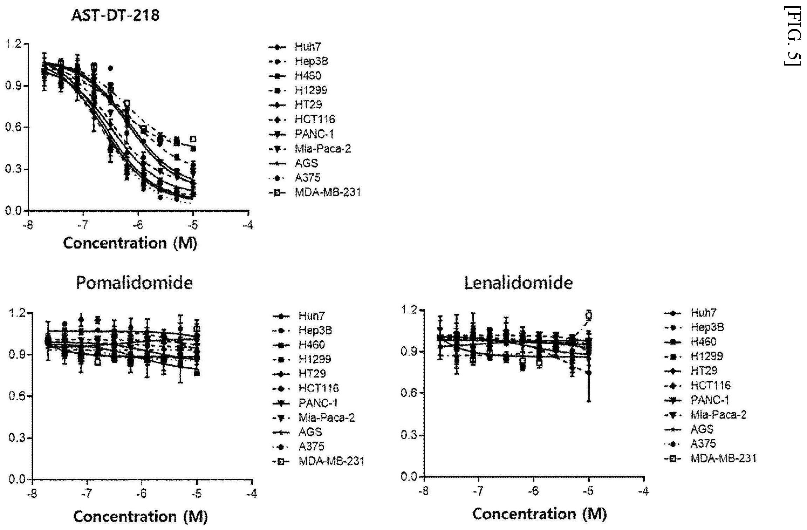

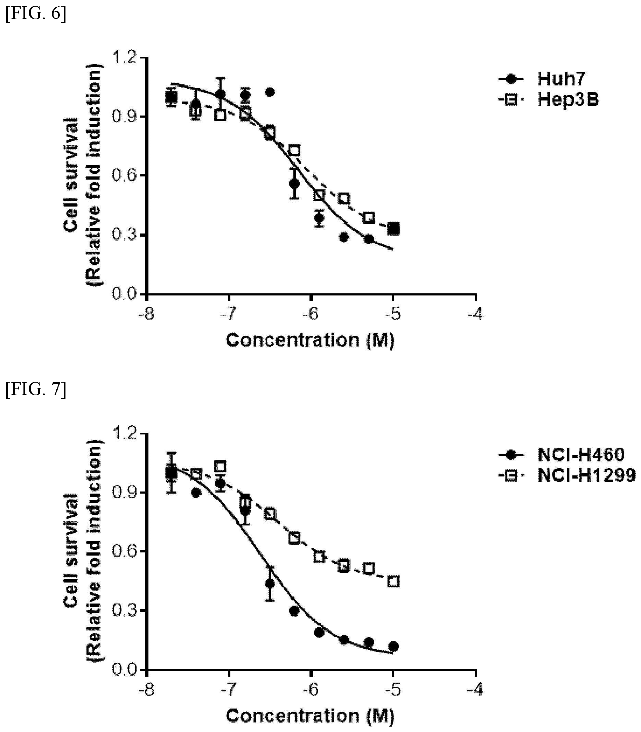

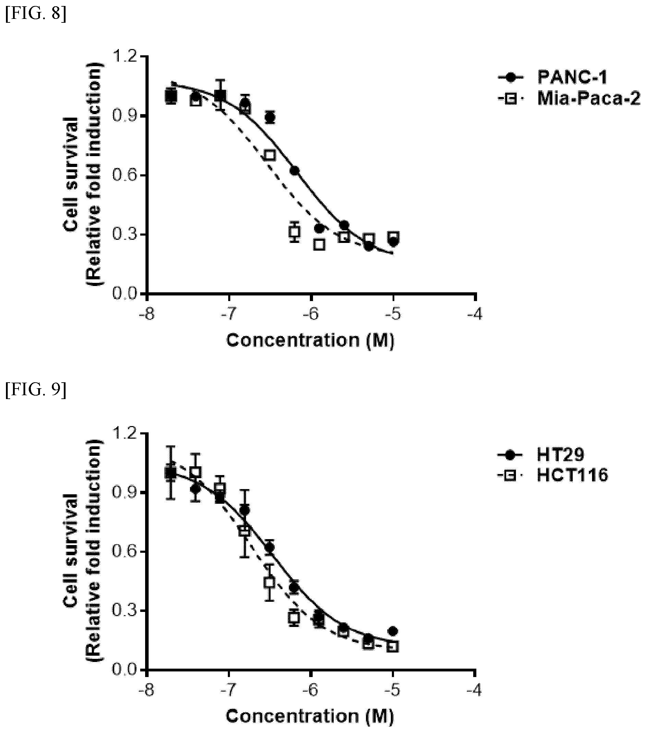

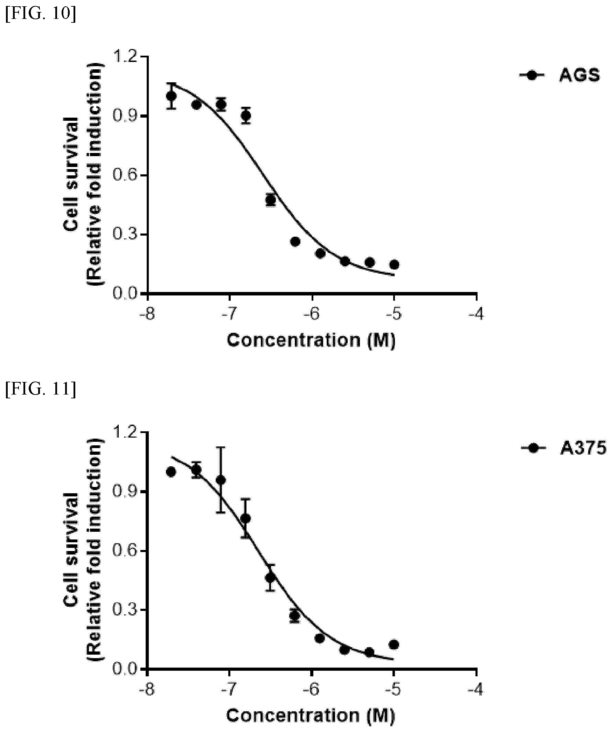

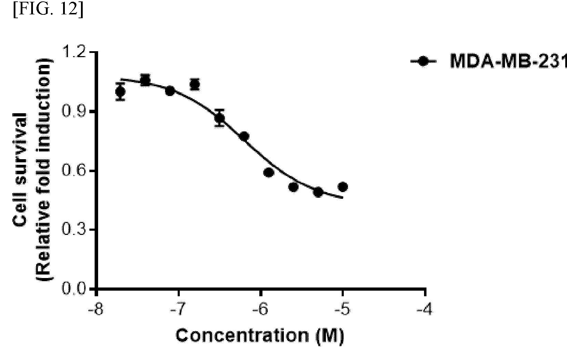

A DRC was created to determine whether AST-DT-218 is effective in killing various cancer cells. First, hepatoma cell lines (Huh7 and Hep3B), non-small cell lung cancer cell lines (NCI-H460 and NCI-H1299), pancreatic cancer cell lines (PANC-1 and Mia-Paca-2), rectal cancer cell lines (HT29 and HCT116), a gastric adenocarcinoma cell line (AGS), a malignant melanoma cell line (A375), and an adenocarcinoma cell line (MDA-MB-231) were prepared. Each cell line was cultured in a flask, and the cells which reached the logarithmic growth phase were prepared, washed with PBS, harvested with trypsin, centrifuged to adjust the cell density, and then resuspended in an appropriate culture medium, and plated into 96-well plates. After culturing until the cells attached to the plates and stabilized, AST-DT-218 and controls (lenalidomide and pomalidomide) were prepared and diluted to various concentrations (10-point by diluting by half from the highest concentration of 50 µM). The wells of the plates were treated with each compound at different concentrations for 48 hours, and the cells were cultured to allow them to react with the compounds. After completion of the culture, cell survival was assessed using a cell counting method through cell nuclear staining. Data were collected by measuring cell survival at each concentration, and then DRC was created (see FIGS. 5 to 12) and IC₅₀ values were calculated (see Table 8).

**[Table 8]**

| Disease | Cell Line | IC₅₀ of AST-DT-218 (µM) |
|---|---|---|
| Hepatocellular Carcinoma | Huh7 | 0.6926 |
| | Hep3B | 0.8823 |
| Non-Small Cell Lung Cancer | NCI-H460 | 0.2416 |
| | NCI-H1299 | 0.3902 |
| Pancreatic Carcinoma | PANC-1 | 0.674 |
| | Mia-Paca-2 | 0.2919 |
| Colorectal Carcinoma | HT29 | 0.3399 |
| | HCT116 | 0.1868 |
| Gastric Adenocarcinoma | AGS | 0.2535 |
| Malignant Melanoma | A375 | 0.2273 |
| Adenocarcinoma | MDA-MB-231 | 0.636 |

Compared to lenalidomide and pomalidomide, AST-DT-218 was confirmed to be effective in killing various cancer cells used in *in vitro* experiments (FIG. 5).

### Experimental Example 7. CRBN Affinity Test

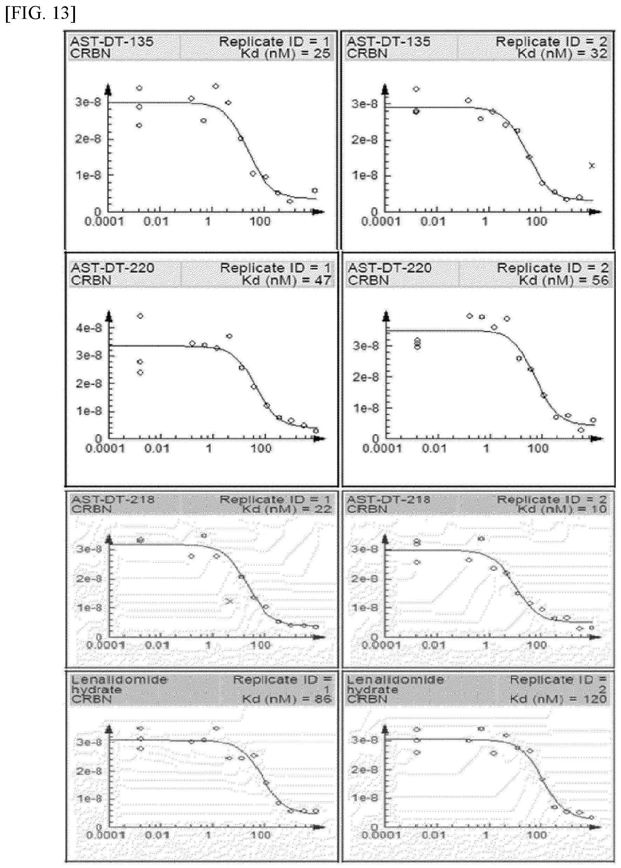

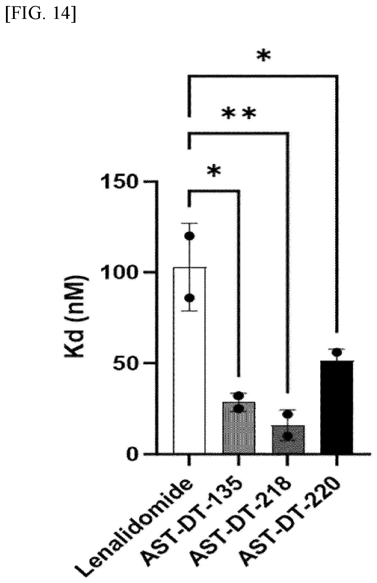

CRBN affinity experiments were performed to evaluate the binding affinity of AST-DT-135, AST-DT-218, and AST-DT-220 to CRBN proteins. First, CRBN proteins were expressed in *E. coli* and purified, and then the purified CRBN proteins were dissolved in a buffer solution (PBS). AST-DT-135, AST-DT-218, AST-DT-220, and lenalidomide hydrate were each dissolved in a solvent, and the CRBN proteins were dispensed into a 96-well plate, and then the CRBN proteins and AST-DT-135, AST-DT-218, AST-DT-220, and a lenalidomide hydrate were each mixed and reacted. After the mixture was sufficiently mixed and reacted, the binding strength at each concentration was measured to collect data, and a binding curve was created therefrom (see FIG. 13), and the KD (equilibrium dissociation constant) values were derived therefrom (see Table 9 and FIG. 14).

**[Table 9]**

| Compound | Kd (nM) on CRBN |
|---|---|
| AST-DT-135 | 29 |
| AST-DT-218 | 16 |
| AST-DT-220 | 52 |
| Lenalidomide Hydrate | 100 |

It was confirmed that the compound of Example of the present invention described above has a much improved binding affinity for the CRBN substrate compared to lenalidomide.

## Claims

1. A compound represented by Formula 1 below, or an enantiomeric compound thereof: 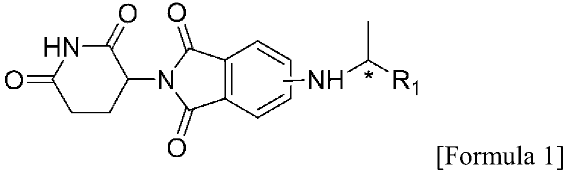
wherein in Formula 1 above,
the R₁ is alkyl, saturated or unsaturated cycloalkyl, aromatic or non-aromatic heterocyclyl, aryl, or -alkyl-C(=O)-O-alkyl;
the alkyl is unsubstituted or substituted with one or more alkoxy groups or hydroxy groups; and
the heterocyclyl or aryl is unsubstituted or substituted with one or more alkyl groups, hydroxy groups, halogens, alkoxy groups, -(C=O)-O-alkyl, -CF₃, -OCF₃, -S-alkyl, -S(O₂)-alkyl, or -S(O₂)NH₂.

2. The compound of claim 1, wherein the R₁ is C₁₋₇ alkyl, C₃₋₇ cycloalkyl, 5- to 12-membered aromatic or non-aromatic heterocyclyl comprising a heteroatom selected from the group consisting of N, O, and S, or C₆₋₁₂ aryl.

3. The compound of claim 1, wherein the R₁ is substituted or unsubstituted C₁₋₅ alkyl, substituted or unsubstituted C₃₋₅ cycloalkyl, substituted or unsubstituted dihydrobenzofuran or pyridine, substituted or unsubstituted phenyl, or C₁₋₅ alkyl-(C=O)-O-alkyl.

4. The compound of claim 1, wherein the R₁ is C₁₋₃ alkyl substituted with a hydroxy group; unsubstituted C₃₋₅ alkyl; or a phenyl substituted with a methoxy group.

5. The compound of claim 1, wherein the compound represented by Formula 1 above is any one selected from the group consisting of the following:
1) methyl (3R)-3-((2-(2,6-dioxopiperidin-3-yl)-1,3-dioxoisoindolin-4-yl)amino)butanoate;
2) methyl (3S)-3-((2-(2,6-dioxopiperidin-3-yl)-1,3-dioxoisoindolin-4-yl)amino)butanoate;
3) 2-(2,6-dioxopiperidin-3-yl)-4-(((S)-1-hydroxypropan-2-yl)amino)isoindoline-1,3-dione;
4) 2-(2,6-dioxopiperidin-3-yl)-4-(((R)-1-hydroxypropan-2-yl)amino)isoindoline-1,3-dione;
5) 2-(2,6-dioxopiperidin-3-yl)-4-(((S)-pentan-2-yl)amino)isoindoline-1,3-dione;
6) 2-(2,6-dioxopiperidin-3-yl)-4-((3-methylbutan-2-yl)amino)isoindoline-1,3-dione;
7) 4-((3,3-dimethylbutan-2-yl)amino)-2-(2,6-dioxopiperidin-3-yl)isoindoline-1,3-dione;
8) 2-(2,6-dioxopiperidin-3-yl)-4-(((S)-1-methoxypropan-2-yl)amino)isoindoline-1,3-dione;
9) 2-(2,6-dioxopiperidin-3-yl)-4-(((R)-1-(4-fluorophenyl)ethyl)amino)isoindoline-1,3-dione;
10) 2-(2,6-dioxopiperidin-3-yl)-4-(((S)-1-(4-fluorophenyl)ethyl)amino)isoindoline-1,3-dione;
11) 2-(2,6-dioxopiperidin-3-yl)-4-(((S)-1-(4-methoxyphenyl)ethyl)amino)isoindoline-1,3-dione;
12) 2-(2,6-dioxopiperidin-3-yl)-4-(((R)-1-(4-methoxyphenyl)ethyl)amino)isoindoline-1,3-dione;
13) 2-(2,6-dioxopiperidin-3-yl)-4-(((R)-1-(4-(trifluoromethoxy)phenyl)ethyl)amino)isoindoline-1,3-dione;
14) 2-(2,6-dioxopiperidin-3-yl)-4-(((S)-1-(4-(trifluoromethoxy)phenyl)ethyl)amino)isoindoline-1,3-dione;
15) 2-(2,6-dioxopiperidin-3-yl)-4-(((R)-1-(2-fluorophenyl)ethyl)amino)isoindoline-1,3-dione;
16) 2-(2,6-dioxopiperidin-3-yl)-4-(((S)-1-(2-fluorophenyl)ethyl)amino)isoindoline-1,3-dione;
17) 2-(2,6-dioxopiperidin-3-yl)-4-(((R)-1-(3-methoxyphenyl)ethyl)amino)isoindoline-1,3-dione;
18) 2-(2,6-dioxopiperidin-3-yl)-4-(((S)-1-(3-methoxyphenyl)ethyl)amino)isoindoline-1,3-dione;
19) 2-(2,6-dioxopiperidin-3-yl)-4-(((R)-1-(2-(trifluoromethyl)phenyl)ethyl)amino)isoindoline-1,3-dione;
20) 4-((1R)-1-((2-(2,6-dioxopiperidin-3-yl)-1,3-dioxoisoindolin-4-yl)amino)ethyl)benzenesulfonamide;
21) methyl 4-((1R)-1-((2-(2,6-dioxopiperidin-3-yl)-1,3-dioxoisoindolin-4-yl)amino)ethyl)benzoate;
22) methyl 4-((1S)-1-((2-(2,6-dioxopiperidin-3-yl)-1,3-dioxoisoindolin-4-yl)amino)ethyl)benzoate;
23) 2-(2,6-dioxopiperidin-3-yl)-4-(((R)-1-(p-tolyl)ethyl)amino)isoindoline-1,3-dione;
24) 2-(2,6-dioxopiperidin-3-yl)-4-(((S)-1-(p-tolyl)ethyl)amino)isoindoline-1,3-dione;
25) 4-(((R)-1-(4-chlorophenyl)ethyl)amino)-2-(2,6-dioxopiperidin-3-yl)isoindoline-1,3-dione;
26) 4-(((S)-1-(4-chlorophenyl)ethyl)amino)-2-(2,6-dioxopiperidin-3-yl)isoindoline-1,3-dione;
27) 2-(2,6-dioxopiperidin-3-yl)-4-(((R)-1-(2-fluoro-4-methoxyphenyl)ethyl)amino)isoindoline-1,3-dione;
28) 2-(2,6-dioxopiperidin-3-yl)-4-(((S)-1-(2-fluoro-4-methoxyphenyl)ethyl)amino)isoindoline-1,3-dione;
29) 2-(2,6-dioxopiperidin-3-yl)-4-(((R)-1-(4-fluoro-3-methoxyphenyl)ethyl)amino)isoindoline-1,3-dione;
30) 2-(2,6-dioxopiperidin-3-yl)-4-(((S)-1-(4-fluoro-3-methoxyphenyl)ethyl)amino)isoindoline-1,3-dione;
31) 2-(2,6-dioxopiperidin-3-yl)-4-(((R)-1-(4-(trifluoromethyl)phenyl)ethyl)amino)isoindoline-1,3-dione;
32) 2-(2,6-dioxopiperidin-3-yl)-4-(((S)-1-(4-(trifluoromethyl)phenyl)ethyl)amino)isoindoline-1,3-dione;
33) 2-(2,6-dioxopiperidin-3-yl)-4-(((R)-1-(4-(methylthio)phenyl)ethyl)amino)isoindoline-1,3-dione;
34) 2-(2,6-dioxopiperidin-3-yl)-4-(((S)-1-(4-(methylthio)phenyl)ethyl)amino)isoindoline-1,3-dione;
35) 2-(2,6-dioxopiperidin-3-yl)-4-(((S)-1-(4-ethoxyphenyl)ethyl)amino)isoindoline-1,3-dione;
36) 2-(2,6-dioxopiperidin-3-yl)-4-(((R)-1-(3-fluorophenyl)ethyl)amino)isoindoline-1,3-dione;
37) 2-(2,6-dioxopiperidin-3-yl)-4-(((S)-1-(3-fluorophenyl)ethyl)amino)isoindoline-1,3-dione;
38) 2-(2,6-dioxopiperidin-3-yl)-4-(((R)-1-(3-fluoro-4-methoxyphenyl)ethyl)amino)isoindoline-1,3-dione;
39) 4-(((R)-1-(3,4-difluorophenyl)ethyl)amino)-2-(2,6-dioxopiperidin-3-yl)isoindoline-1,3-dione;
40) 4-(((S)-1-(3,4-difluorophenyl)ethyl)amino)-2-(2,6-dioxopiperidin-3-yl)isoindoline-1,3-dione;
41) 4-(((R)-1-(3,5-difluorophenyl)ethyl)amino)-2-(2,6-dioxopiperidin-3-yl)isoindoline-1,3-dione;
42) 4-(((S)-1-(3,5-difluorophenyl)ethyl)amino)-2-(2,6-dioxopiperidin-3-yl)isoindoline-1,3-dione;
43) 2-(2,6-dioxopiperidin-3-yl)-4-(((R)-1-(6-methoxypyridin-3-yl)ethyl)amino)isoindoline-1,3-dione;
44) 2-(2,6-dioxopiperidin-3-yl)-4-(((S)-1-(6-methoxypyridin-3-yl)ethyl)amino)isoindoline-1,3-dione;
45) 4-(((R)-1-(3,5-dimethoxyphenyl)ethyl)amino)-2-(2,6-dioxopiperidin-3-yl)isoindoline-1,3-dione;
46) 2-(2,6-dioxopiperidin-3-yl)-4-(((S)-1-(3-fluoro-4-methoxyphenyl)ethyl)amino)isoindoline-1,3-dione;
47) 2-(2,6-dioxopiperidin-3-yl)-4-(((R)-1-(5-methoxypyridin-2-yl)ethyl)amino)isoindoline-1,3-dione;
48) 2-(2,6-dioxopiperidin-3-yl)-4-(((S)-1-(5-methoxypyridin-2-yl)ethyl)amino)isoindoline-1,3-dione;
49) 2-(2,6-dioxopiperidin-3-yl)-4-(((R)-1-(4-(methylsulfonyl)phenyl)ethyl)amino)isoindoline-1,3-dione;
50) 2-(2,6-dioxopiperidin-3-yl)-4-(((S)-1-(4-(methylsulfonyl)phenyl)ethyl)amino)isoindoline-1,3-dione;
51) 4-(((R)-1-(2,3-dihydrobenzofuran-5-yl)ethyl)amino)-2-(2,6-dioxopiperidin-3-yl)isoindoline-1,3-dione; and
52) 4-(((S)-1-cyclopropylethyl)amino)-2-(2,6-dioxopiperidin-3-yl)isoindoline-1,3-dione.

6. The compound of claim 1, wherein the compound represented by Formula 1 is 2-(2,6-dioxopiperidin-3-yl)-4-(((R)-1-hydroxypropan-2-yl)amino)isoindoline-1,3-dione; 2-(2,6-dioxopiperidin-3-yl)-4-(((S)-pentan-2-yl)amino)isoindoline-1,3-dione; or 2-(2,6-dioxopiperidin-3-yl)-4-(((R)-1-(4-methoxyphenyl)ethyl)amino)isoindoline-1,3-dione.

7. A pharmaceutical composition for preventing or treating cancer, comprising a compound represented by Formula 1, or an enantiomeric compound thereof of claim 1 as an active ingredient,
wherein the cancer is any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.

8. The pharmaceutical composition of claim 7, wherein the cancer is recurrent or incurable cancer.

9. The pharmaceutical composition of claim 7, wherein the cancer is a drugresistant cancer.

10. The pharmaceutical composition of claim 7, wherein the active ingredient is any one selected from the group consisting of the following:
1) methyl (3R)-3-((2-(2,6-dioxopiperidin-3-yl)-1,3-dioxoisoindolin-4-yl)amino)butanoate;
2) methyl (3S)-3-((2-(2,6-dioxopiperidin-3-yl)-1,3-dioxoisoindolin-4-yl)amino)butanoate;
3) 2-(2,6-dioxopiperidin-3-yl)-4-(((S)-1-hydroxypropan-2-yl)amino)isoindoline-1,3-dione;
4) 2-(2,6-dioxopiperidin-3-yl)-4-(((R)-1-hydroxypropan-2-yl)amino)isoindoline-1,3-dione;
5) 2-(2,6-dioxopiperidin-3-yl)-4-(((S)-pentan-2-yl)amino)isoindoline-1,3-dione;
6) 2-(2,6-dioxopiperidin-3-yl)-4-((3-methylbutan-2-yl)amino)isoindoline-1,3-dione;
7) 4-((3,3-dimethylbutan-2-yl)amino)-2-(2,6-dioxopiperidin-3-yl)isoindoline-1,3-dione;
8) 2-(2,6-dioxopiperidin-3-yl)-4-(((S)-1-methoxypropan-2-yl)amino)isoindoline-1,3-dione;
9) 2-(2,6-dioxopiperidin-3-yl)-4-(((R)-1-(4-fluorophenyl)ethyl)amino)isoindoline-1,3-dione;
10) 2-(2,6-dioxopiperidin-3-yl)-4-(((S)-1-(4-fluorophenyl)ethyl)amino)isoindoline-1,3-dione;
11) 2-(2,6-dioxopiperidin-3-yl)-4-(((S)-1-(4-methoxyphenyl)ethyl)amino)isoindoline-1,3-dione;
12) 2-(2,6-dioxopiperidin-3-yl)-4-(((R)-1-(4-methoxyphenyl)ethyl)amino)isoindoline-1,3-dione;
13) 2-(2,6-dioxopiperidin-3-yl)-4-(((R)-1-(4-(trifluoromethoxy)phenyl)ethyl)amino)isoindoline-1,3-dione;
14) 2-(2,6-dioxopiperidin-3-yl)-4-(((S)-1-(4-(trifluoromethoxy)phenyl)ethyl)amino)isoindoline-1,3-dione;
15) 2-(2,6-dioxopiperidin-3-yl)-4-(((R)-1-(2-fluorophenyl)ethyl)amino)isoindoline-1,3-dione;
16) 2-(2,6-dioxopiperidin-3-yl)-4-(((S)-1-(2-fluorophenyl)ethyl)amino)isoindoline-1,3-dione;
17) 2-(2,6-dioxopiperidin-3-yl)-4-(((R)-1-(3-methoxyphenyl)ethyl)amino)isoindoline-1,3-dione;
18) 2-(2,6-dioxopiperidin-3-yl)-4-(((S)-1-(3-methoxyphenyl)ethyl)amino)isoindoline-1,3-dione;
19) 2-(2,6-dioxopiperidin-3-yl)-4-(((R)-1-(2-(trifluoromethyl)phenyl)ethyl)amino)isoindoline-1,3-dione;
20) 4-((1R)-1-((2-(2,6-dioxopiperidin-3-yl)-1,3-dioxoisoindolin-4-yl)amino)ethyl)benzenesulfonamide;
21) methyl 4-((1R)-1-((2-(2,6-dioxopiperidin-3-yl)-1,3-dioxoisoindolin-4-yl)amino)ethyl)benzoate;
22) methyl 4-((1S)-1-((2-(2,6-dioxopiperidin-3-yl)-1,3-dioxoisoindolin-4-yl)amino)ethyl)benzoate;
23) 2-(2,6-dioxopiperidin-3-yl)-4-(((R)-1-(p-tolyl)ethyl)amino)isoindoline-1,3-dione;
24) 2-(2,6-dioxopiperidin-3-yl)-4-(((S)-1-(p-tolyl)ethyl)amino)isoindoline-1,3-dione;
25) 4-(((R)-1-(4-chlorophenyl)ethyl)amino)-2-(2,6-dioxopiperidin-3-yl)isoindoline-1,3-dione;
26) 4-(((S)-1-(4-chlorophenyl)ethyl)amino)-2-(2,6-dioxopiperidin-3-yl)isoindoline-1,3-dione;
27) 2-(2,6-dioxopiperidin-3-yl)-4-(((R)-1-(2-fluoro-4-methoxyphenyl)ethyl)amino)isoindoline-1,3-dione;
28) 2-(2,6-dioxopiperidin-3-yl)-4-(((S)-1-(2-fluoro-4-methoxyphenyl)ethyl)amino)isoindoline-1,3-dione;
29) 2-(2,6-dioxopiperidin-3-yl)-4-(((R)-1-(4-fluoro-3-methoxyphenyl)ethyl)amino)isoindoline-1,3-dione;
30) 2-(2,6-dioxopiperidin-3-yl)-4-(((S)-1-(4-fluoro-3-methoxyphenyl)ethyl)amino)isoindoline-1,3-dione;
31) 2-(2,6-dioxopiperidin-3-yl)-4-(((R)-1-(4-(trifluoromethyl)phenyl)ethyl)amino)isoindoline-1,3-dione;
32) 2-(2,6-dioxopiperidin-3-yl)-4-(((S)-1-(4-(trifluoromethyl)phenyl)ethyl)amino)isoindoline-1,3-dione;
33) 2-(2,6-dioxopiperidin-3-yl)-4-(((R)-1-(4-(methylthio)phenyl)ethyl)amino)isoindoline-1,3-dione;
34) 2-(2,6-dioxopiperidin-3-yl)-4-(((S)-1-(4-(methylthio)phenyl)ethyl)amino)isoindoline-1,3-dione;
35) 2-(2,6-dioxopiperidin-3-yl)-4-(((S)-1-(4-ethoxyphenyl)ethyl)amino)isoindoline-1,3-dione;
36) 2-(2,6-dioxopiperidin-3-yl)-4-(((R)-1-(3-fluorophenyl)ethyl)amino)isoindoline-1,3-dione;
37) 2-(2,6-dioxopiperidin-3-yl)-4-(((S)-1-(3-fluorophenyl)ethyl)amino)isoindoline-1,3-dione;
38) 2-(2,6-dioxopiperidin-3-yl)-4-(((R)-1-(3-fluoro-4-methoxyphenyl)ethyl)amino)isoindoline-1,3-dione;
39) 4-(((R)-1-(3,4-difluorophenyl)ethyl)amino)-2-(2,6-dioxopiperidin-3-yl)isoindoline-1,3-dione;
40) 4-(((S)-1-(3,4-difluorophenyl)ethyl)amino)-2-(2,6-dioxopiperidin-3-yl)isoindoline-1,3-dione;
41) 4-(((R)-1-(3,5-difluorophenyl)ethyl)amino)-2-(2,6-dioxopiperidin-3-yl)isoindoline-1,3-dione;
42) 4-(((S)-1-(3,5-difluorophenyl)ethyl)amino)-2-(2,6-dioxopiperidin-3-yl)isoindoline-1,3-dione;
43) 2-(2,6-dioxopiperidin-3-yl)-4-(((R)-1-(6-methoxypyridin-3-yl)ethyl)amino)isoindoline-1,3-dione;
44) 2-(2,6-dioxopiperidin-3-yl)-4-(((S)-1-(6-methoxypyridin-3-yl)ethyl)amino)isoindoline-1,3-dione;
45) 4-(((R)-1-(3,5-dimethoxyphenyl)ethyl)amino)-2-(2,6-dioxopiperidin-3-yl)isoindoline-1,3-dione;
46) 2-(2,6-dioxopiperidin-3-yl)-4-(((S)-1-(3-fluoro-4-methoxyphenyl)ethyl)amino)isoindoline-1,3-dione;
47) 2-(2,6-dioxopiperidin-3-yl)-4-(((R)-1-(5-methoxypyridin-2-yl)ethyl)amino)isoindoline-1,3-dione;
48) 2-(2,6-dioxopiperidin-3-yl)-4-(((S)-1-(5-methoxypyridin-2-yl)ethyl)amino)isoindoline-1,3-dione;
49) 2-(2,6-dioxopiperidin-3-yl)-4-(((R)-1-(4-(methylsulfonyl)phenyl)ethyl)amino)isoindoline-1,3-dione;
50) 2-(2,6-dioxopiperidin-3-yl)-4-(((S)-1-(4-(methylsulfonyl)phenyl)ethyl)amino)isoindoline-1,3-dione;
51) 4-(((R)-1-(2,3-dihydrobenzofuran-5-yl)ethyl)amino)-2-(2,6-dioxopiperidin-3-yl)isoindoline-1,3-dione; and
52) 4-(((S)-1-cyclopropylethyl)amino)-2-(2,6-dioxopiperidin-3-yl)isoindoline-1,3-dione.

11. The pharmaceutical composition of claim 7, wherein the active ingredient is 2-(2,6-dioxopiperidin-3-yl)-4-(((R)-1-hydroxypropan-2-yl)amino)isoindoline-1,3-dione; 2-(2,6-dioxopiperidin-3-yl)-4-(((S)-pentan-2-yl)amino)isoindoline-1,3-dione; or 2-(2,6-dioxopiperidin-3-yl)-4-(((R)-1-(4-methoxyphenyl)ethyl)amino)isoindoline-1,3-dione.

12. The pharmaceutical composition of claim 7, wherein the composition is administered by intraperitoneal administration, intravenous administration, intramuscular administration, subcutaneous administration, intradermal injection, oral administration, topical administration, intranasal injection, intrapulmonary injection, or rectal administration.

13. A method for preventing or treating cancer in a subject, comprising administering, to a subject, a compound represented by Formula 1, or an enantiomeric compound thereof of claim 1,
wherein the cancer is any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.

14. Use of a compound represented by Formula 1, or an enantiomeric compound thereof of claim 1, for the preparation of a medicament for preventing or treating cancer, wherein the cancer is any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.

15. Use of a compound represented by Formula 1, or an enantiomeric compound thereof of claim 1, for preventing or treating cancer,
wherein the cancer is any one selected from the group consisting of liver cancer, non-small cell lung cancer, lung cancer, pancreatic cancer, rectal cancer, gastric adenocarcinoma, stomach cancer, malignant melanoma, breast cancer, multiple myeloma, blood cancer, and adenocarcinoma.
